# EUROPEAN PATENT SPECIFICATION

(11) **EP 2 852 617 B1**
(45) Date of publication and mention of the grant of the patent: **12.12.2018**
(21) Application number: 13711510.1
(22) Date of filing: 06.03.2013
(51) Int. Cl.: C07K 16/00, C12N 5/0783, C07K 16/28

(54) **RECOMBINANT ANTIBODY COMPOSITIONS AND METHODS OF USE THEREOF**
REKOMBINANTE ANTIKÖRPERZUSAMMENSETZUNGEN UND ANWENDUNGSVERFAHREN DAFÜR
COMPOSITIONS D'ANTICORPS RECOMBINANT ET LEURS PROCÉDÉS D'UTILISATION

(30) Priority: 22.05.2012 US 201261650388 P
(43) Date of publication of application: 01.04.2015
(73) Proprietor: Life Technologies AS, 0379 Oslo (NO)
(72) Inventor: SCHJETNE, Karoline, Carlsbad, CA 92008 (US); AARVAK, Tanja, Carlsbad, CA 92008 (US); NORDERHAUG, Lars, Carlsbad, CA 92008 (US)
(74) Representative: Thermo Fisher Scientific- Life Sciences Solutions Group
(86) International application number: PCT/US2013/029450
(87) International publication number: WO 2013/176730

(56) References cited:
- WO-A1-03/040355
- US-A1- 2008 255 004
- B. W. Senior ET AL: "Cleavage of a Recombinant Human Immunoglobulin A2 (IgA2)-IgA1 Hybrid Antibody by Certain Bacterial IgA1 Proteases", Infection and Immunity, vol. 68, no. 2, 1 February 2000 (2000-02-01), pages 463-469, XP055261844, US ISSN: 0019-9567, DOI: 10.1128/IAI.68.2.463-469.2000
- B. W. Senior ET AL: "The Influences of Hinge Length and Composition on the Susceptibility of Human IgA to Cleavage by Diverse Bacterial IgA1 Proteases", THE JOURNAL OF IMMUNOLOGY, vol. 174, no. 12, 8 June 2005 (2005-06-08) , pages 7792-7799, XP055261847, US ISSN: 0022-1767, DOI: 10.4049/jimmunol.174.12.7792

## Description

### CROSS REFERENCE TO RELATED APPLICATIONS

This application claims the benefit under 35 U.S.C. 119(e) of U.S. Provisional Patent Application No. 61/650,388, filed May 22, 2012.

### TECHNICAL FIELD

The present invention relates to recombinant antibodies and their use in methods for isolating or otherwise preparing a population of target cells. In particular embodiments, it relates to recombinant antibodies comprising an exogenous proteolytic cleavage site and nucleic acid molecules encoding the antibodies.

### BACKGROUND

Isolated cells or cell compositions enriched for particular cells have long been of great interest and utility to the research and clinical communities and to the pharmaceutical industry. Accordingly, different procedures and reagents have been developed to prepare such isolated or enriched cell compositions from a less pure or mixed cell population. Various cell attributes have be used to segregate a particular cell from a mixed cell population including the size, shape, motility, and surface molecules of the target cell.

Antibodies which specifically associate with target molecules on the surface of the cells of interest have been used to separate the desired (or target) cells from other cells of a mixed population which do not have the particular surface molecule. For example, antibodies specific for particular cell surface molecules have been used to label cells of interest and then to positively or negatively select the labeled cells to prepare a desired cell population. Senior et al. Infection and Immunity, 2000, Vol. 68 p. 463-469 and Senior and Woof, Immunol 2005; Vol. 174, p.7792-7799, disclose antibodies with a swapped native hinge region or fragments of a native hinge region in order to analyse their susceptibility to bacterial proteases. Those protease cleavage sites are naturally occurring and the antibodies having these engineered cleavage sites are not used in methods for isolating or activating cells by using the antibody as a cleavable bridge between cell and solid support.

Antibody-coated surfaces, such as beads or culture wells, have been used to capture specific cells and to separate the bound cells from the unbound cells in the population. However, many uses for the isolated cells require removal of the cells from the capture antibody/surface complex and this removal can result in cell damage or alteration that leaves the isolated cells unsuitable for or incompatible with particular uses or downstream applications.

For example, methods which use a cocktail of proteolytic and collagenolytic enzymes to remove cells from a capture antibody/bead complex can degrade many cell surface molecules and alter at least the surface characteristics of the isolated cells. In other methods, cells are released from a capture antibody/bead complex by separating the capture antibody from the bead but leaving the capture antibody bound to the target cell surface. While still other cell isolation methods simply do not remove the capture antibody/bead complex from the isolated cells.

There remains a need for alternative procedures and reagents for cell isolation that, for example, produce specific cell populations without carrying over isolation reagents or modifying the cells in ways which may be undesirable for uses and further applications of the cells.

### SUMMARY

Provided herein, in part, are methods for isolating a target cell from a mixed population using a recombinant antibody which specifically binds the target cell and a protease which cleaves an exogenous proteolytic cleavage site in the antibody. Also provided is a recombinant antibody comprising an exogenous proteolytic cleavage site, as well as compositions comprising the antibody. Also provided is a nucleic acid molecule and expression vector encoding the antibody.

In one aspect, provided herein is a method for detaching a target cell attached to a recombinant antibody-coated solid surface, the method comprising contacting the target cell attached to the surface with a protease, or portion of a protease, specific for an exogenous proteolytic cleavage site in the antibody, thereby detaching the target cell from the surface.

In another aspect, provided herein is a method for isolating a target cell from a mixed cell population, the method comprising a) contacting a mixed cell population with an antibody attached to a solid surface, wherein the antibody specifically binds to the target cell in the population and wherein the antibody comprises an exogenous proteolytic cleavage site. In some embodiments, the method further comprises b) separating the target cells bound to the surface from cells not bound to the surface; optionally further comprising c) contacting the target cells bound to the surface with a protease, or portion of a protease, specific for the cleavage site to detach the target cells from the surface; and optionally further comprising d) collecting the target cells detached from the surface.

In another aspect, provided herein is a method for producing a population of activated target cells, the method comprising a) contacting a cell population comprising target cells with an agonistic antibody attached to a solid surface, wherein the antibody specifically binds to the target cells in the population and wherein the antibody comprises an exogenous proteolytic cleavage site; and b) incubating the cells and the antibody attached to the surface under conditions to allow activation of the target cells. In some embodiments, the method further comprises c) separating the target cells bound to the surface from cells not bound to the surface; optionally further comprising d) contacting the target cells bound to the surface with a protease, or portion of a protease, specific for the cleavage site to detach the target cells from the surface; and optionally further comprising e) collecting the activated target cells detached from the surface.

In another aspect, provided herein is a method for positively isolating an antibody-free population of target cells, the method comprising: a) contacting a mixed cell population with an antibody attached to a solid surface, wherein the antibody specifically binds to the target cell in the population and wherein the antibody comprises an exogenous protease cleavage site; b) separating the target cells bound to the surface from cells not bound to the surface; c) contacting the target cells bound to the surface with a protease, or portion of a protease, specific for the cleavage site to detach the target cells from the surface; and d) collecting the target cells detached from the antibody and the surface to form a population of target cells free of bound antibody.

In another aspect, provided herein is a method for producing a bead-free population of target cells, the method comprising a) contacting a mixed cell population with an antibody attached to beads, wherein the antibody specifically binds to the target cell in the population and wherein the antibody comprises an exogenous protease cleavage site; b) separating the target cells bound to the beads from cells not bound to the beads; c) contacting the target cells bound to the beads with a protease, or portion of a protease, specific for the cleavage site to detach the target cells from the beads; and d) collecting the target cells detached from the beads to form a bead-free population of target cells.

In the methods of the invention the recombinant antibody comprises an exogenous proteolytic cleavage site for a protease selected from the group consisting of a tobacco etch virus (TEV) protease, a rhinovirus 3C protease, a TVMV protease, a plum pox virus protease, a turnip mosaic virus protease, enteropeptidase, thrombin and Factor Xa in the hinge region.

In another aspect, provided herein is a composition comprising a cell, a recombinant antibody, and a solid surface, where the recombinant antibody is specifically bound to the cell and is also attached to the solid surface, and where the recombinant antibody comprises an exogenous proteolytic cleavage site.

In another aspect, provided herein is a nucleic acid molecule encoding an antibody heavy chain comprising an exogenous proteolytic cleavage site in the hinge region.

In some embodiments, the protease is not expressed by the target cell.

In some embodiments, the protease is a viral protease. In certain embodiments, the protease is selected from the group consisting of a tobacco etch virus (TEV) protease, a rhinovirus 3C protease, a TVMV protease, a plum pox virus protease, and a turnip mosaic virus protease.

In some embodiments, the protease is an endoprotease. In certain embodiments, the protease is selected from the group consisting of enteropeptidase, thrombin and Factor Xa.

In some embodiments of the provided methods and compositions, the exogenous proteolytic cleavage site is for a protease not expressed in the target cell.

In some embodiments of the provided methods and compositions, the exogenous proteolytic cleavage site is for a viral protease. In certain embodiments, the exogenous proteolytic cleavage site is for a protease selected from the group consisting of a tobacco etch virus (TEV) protease, a rhinovirus 3C protease, a TVMV protease, a plum pox virus protease, and a turnip mosaic virus protease.

In some embodiments of the provided methods and compositions, the exogenous proteolytic cleavage site is for an endoprotease. In certain embodiments, the exogenous proteolytic cleavage site is for a protease is selected from the group consisting of enteropeptidase, thrombin and Factor Xa.

In some embodiments, the exogenous proteolytic cleavage site is in the upper hinge region of the antibody.

In some embodiments, the target cell is a T cell, a stem cell, or a CD34+ cell.

In some embodiments, the recombinant antibody specifically binds to an endogenous protein on the target cell surface. In some embodiments, the recombinant antibody specifically binds to an exogenous protein on the target cell surface. In some embodiments, the recombinant antibody specifically binds a CD3 epitope, a Tra-1-60 epitope, or a CD34 epitope.

In some embodiments, the recombinant antibody is directly attached to a solid surface. In other embodiments, the recombinant antibody is indirectly attached to a solid surface. In some embodiments, the solid surface is a culture well or bead. In certain embodiments, the solid surface is a paramagnetic bead.

### BRIEF DESCRIPTION OF THE DRAWINGS

Fig. 1 A-B depict maps of high expression backbone H-chain (Fig. 1A, mIgG1-TEV SPV) and L-chain (Fig. 1B, mCK-SPV) vectors with V-regions encoding anti-human CD3 and genomic sequence of H- and L-chain genes. Restriction enzyme sites used for V-region or C-region switch are indicated.
Fig. 2 shows electrophoresis of recombinant antibodies (rAb TEV) under non-reduced (left) and reduced (right) conditions along with controls: molecular weight markers (MwM), purified mouse IgG1 (Std IgG1), spent supernatant from mock transfected cells (Neg), and control plasmids expressing human IgG (hIgG).
Fig. 3 A-B depict flow cytometry analysis of mononuclear cell (MNC) staining by anti-CD3-APC with (Fig. 3A) and without (Fig. 3B) prior treatment of the cells with rAb-TEV.
Fig. 4 shows electrophoresis of rAb TEV following incubation with AcTEV™ Protease (lanes 3 and 4) along with controls: molecular weight markers and rAb-TEV under non-reduced conditions (lane 1), rAB-TEV under reduced conditions (lane 2), control IgG with AcTEV™ Protease (lane 5). AcTEV™ Protease is 27 kDa in molecular weight.
Fig. 5 depicts flow cytometry analysis of rAb-TEV coupled to Dynabeads and detected by anti-mouse IgG1-FITC (top panel) along with controls: rAb-TEV coupled to Dynabeads + anti-mouse IgG2a-FITC (bottom left panel) and mock transfected supernantant coupled to Dynabeads + anti-mouse IgG1-FITC (bottom right panel).
Fig. 6 depicts flow cytometry histograms of rAb-TEV coupled to Dynabeads and detected by anti-mouse kappa-PE antibody before (top left) and after (bottom left and right) incubation with AcTEV™ Protease. A cartoon depicting the two scenarios is shown in top right. Incubation with AcTEV™ Protease was at room temperature (bottom left) or at 37oC (bottom right). The percentage of kappa-positive beads is indicated in each histogram.
Fig. 7 is a chart depicting the percentage of depletion of CD3+ T cell from a MNC population using varying amounts of rAb-TEV bound per 10e7 beads.
Fig. 8 is a cartoon depicting an exemplary workflow for positive isolation of bead-free CD3+ T cells using rAb-TEV coupled to magnetic beads and TEV protease for bead release.
Fig. 9 depicts the recovery, purity and viability of isolated CD3+ cells using beads with varying amounts of attached antibody and the control performed without protease.
Fig. 10 is a chart showing the average recovery and purity of positively isolated CD3+ T cells from MNCs of healthy donors (n=7).
Fig. 11 A-C depict results showing release of capture antibody and availability of target epitope on cells following protease treatment.
Fig. 12 depicts results of activation and expansion of positively isolated CD3+ T cells: (Fig. 12A) fold cell expansion at day 6; (Fig. 12B) CD62L and CD27 expression on CD4 and CD8 cells.
Fig. 13 is a cartoon depicting exemplary compositions and use thereof for cell isolation. The scissors represents a protease specific for the exogenous proteolytic cleavage site in the antibody specific for the target cell epitope.
Fig. 14 A-C depict flow cytometry analysis of a mix of KG1a and FreeStyle™ 293F cells stained by anti-CD34-FITC: (Fig. 14A) without prior treatment of the cells with rAb-TEV anti-CD34; (Fig. 14B) with prior treatment of the cells with rAb-TEV anti-CD34; (Fig. 14C) with prior treatment of the cells with rAb-TEV anti-CD34 and AcTEV protease showing release of rAb-TEV anti-CD34 and availability of target epitope on cells following protease treatment.
Fig. 15 shows electrophoresis of rAb TEV anti-CD34 following incubation with AcTEV™ Protease (lanes 3 and 4) along with controls: molecular weight markers (lane 1), rAB-TEV anti-CD34 without protease (lane 2).
Fig. 16 depicts the recovery, depletion, and purity of isolated CD34+ cells using rAb-TEV anti-CD34 conjugated to beads with three different post-bead release wash conditions. The data shown are the average of triplicates.

### DETAILED DESCRIPTION

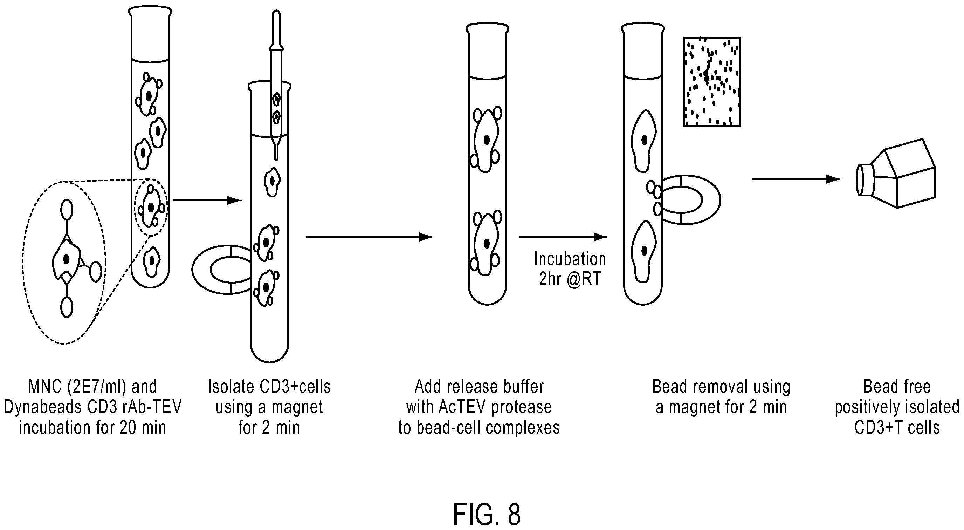

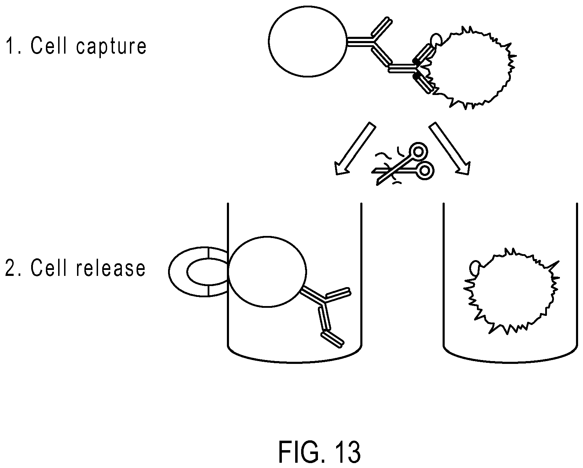

Disclosed herein are methods and compositions which relate, in part, to isolation of a population of target cells. The methods and compositions provided herein can be used to isolate a target cell population without the isolated cells carrying the isolation reagents which may induce or prevent signaling events when the cells are used in further applications. In exemplary embodiments, use of the provided methods and compositions permit the preparation of cells free of the biologically active portion of the capture antibody (the Fc portion) and free of the capture beads enabling the use of such a cell preparation for clinical and in vivo applications. See the illustrations in Figs. 13 and 8 for a summary of several steps of the methods of particular embodiments and for a summary of an exemplary workflow, respectively.

The methods involve the use of an antibody which contains an exogenous proteolytic cleavage site in the hinge region, that is, an antibody which has been modified to include a proteolytic cleavage site that is not naturally found in the antibody. The methods also involve the use a protease with proteolytic activity specific for the exogenous cleavage site inserted in the antibody. The proteolytic cleavage site in the antibody is a site for a protease selected from the group consisting of a tobacco etch virus (TEV) protease, a rhinovirus 3C protease, a TVMV protease, a plum pox virus protease, a turnip mosaic virus protease, enteropeptidase, thrombin and Factor Xa. Generally, as is known in the art, in addition to the amino acids at which the cleavage occurs, a proteolytic cleavage site also includes additional amino acid residues that help to define the protease specificity for the cleavage site.

The recombinant antibody containing the exogenous proteolytic cleavage site binds an epitope on the target cell, that is, the cell that makes up the desired cell population. The recombinant antibody is also referred to herein as the anti-target epitope antibody since it binds an epitope on the target cell.

In one aspect of the methods, a target cell attached to a capture antibody-coated solid surface is detached from the surface by contacting the target cell/surface complex with a protease, where the protease is specific for an exogenous proteolytic cleavage site in the capture antibody. Contact with the protease results in cleavage of exogenous the proteolytic site in the antibody and detaching of the target cell from the surface.

In some embodiments, target cells are isolated from a mixed cell population through use of a recombinant antibody that specifically binds an epitope on the surface of the target cell but not on the surface of non-target cells in the population. Before, during, or after the binding of the target cell epitope by the antibody, the antibody is attached to a surface to enable the separation of the bound target cells from the unbound non-target cells. The recombinant antibody comprises an inserted exogenous proteolytic cleavage site that allows for release or detachment of the cell from the surface through treatment of the cell/antibody/surface complex with a protease specific for the exogenous proteolytic cleavage site. The methods allow for enrichment of target cells or depletion of target cells from a mixed cell population.

In some embodiments of the methods and compositions, the contacting with the protease results in at least 50% of the bound target cells detaching from the surface. In other embodiments, the contacting with the protease results in >60%, >70%, >80%, >90%, or >95% of the bound target cells detaching from the surface.

For example, a method for isolating a target cell from a mixed cell population comprises: a) contacting a mixed cell population with an antibody attached to a solid surface, wherein the antibody specifically binds to the target cell in the population and wherein the antibody comprises an exogenous proteolytic cleavage site; b) separating the target cells bound to the surface from cells not bound to the surface; c) contacting the target cells bound to the surface with a protease, or portion of a protease, specific for the cleavage site to detach the target cells from the surface; and d) collecting the target cells detached from the surface.

In some embodiments, a mixed cell population is incubated with the recombinant anti-target epitope antibody prior to the antibody attaching to a surface. Following the recombinant antibody binding the target cell, the antibody is attached either directly or indirectly to a surface to enable the separation of the bound target cells from the unbound non-target cells. Antibody attachment to the surface following antibody-target cell binding is useful, in particular, for the isolation of a rare cell population, such as, for example, a CD34+ cell population.

Also provided are methods for producing a population of activated target cells, such as, for example, CD3+ T cells which can be activated by incubation with an anti-CD3+ antibody in the presence of IL-2. An example of a method for producing a population of activated target cells comprises: a) contacting a cell population comprising target cells with an agonistic antibody attached to a solid surface, the agonistic antibody specifically binds to the target cells and the antibody comprises an exogenous proteolytic cleavage site; and b) incubating the cells and the antibody attached to the surface under conditions to allow activation of the target cells. The method may optionally further comprise: c) separating the target cells bound to the surface from cells not bound to the surface; d) contacting the target cells bound to the surface with a protease, or portion of a protease, specific for the cleavage site to detach the target cells from the surface; and e) collecting the activated target cells detached from the surface. Such isolated activated cells may be used immediately in particular application or may be expanded prior to use in an application. For example, isolated activated T cells may be expanded 10-fold to 1000-fold in culture prior to use in an in vivo application.

In some embodiments, the exogenous proteolytic cleavage site is inserted into the hinge region of the recombinant antibody. With the exogenous cleavage site in the hinge region, treatment with the specific protease results in cleavage of the recombinant antibody into 3 fragments: 2 Fab fragments and 1 Fc fragment.

The Fc portion of an antibody harbors effector functions and mediates different physiological effects of the immune system in part through binding Fc receptors and other molecules of the immune system. Detaching the target cells from the antibody/surface complex through an exogenous cleavage site which removes the Fc portion of the recombinant antibody from the target cell produces an isolated target cell population free of the Fc domain and its associated effector functions. Accordingly, isolation of target cells using the methods and recombinant antibody provided herein can produce a target cell population free of anti-target epitope antibody Fc domain. For use in cell therapy applications, purification of cells without biologically active antibody (i.e., without the Fc domain) attached is important for the safety and efficacy of the treatments.

The Fab fragments of an antibody are monovalent and bind to the target epitope with lower avidity than the complete antibody, which is at least divalent, binds to the epitope. Thus, the Fab fragments bound to the target epitope on the cell following protease treatment detach from cells more easily than complete antibody. Accordingly, isolation of target cells using the methods and recombinant antibody provided herein can produce an antibody-free target cell population.

In some embodiments, provided is a method for positively isolating an antibody-free population of target cells which comprises: a) contacting a mixed cell population with an antibody attached to a solid surface, the antibody specifically binds to the target cell and the antibody comprises an exogenous proteolytic cleavage site; b) separating the target cells bound to the surface from cells not bound to the surface; c) contacting the surface bound target cells with a protease, or portion of a protease, specific for the exogenous cleavage site in the antibody to detach the target cells from the surface; and d) collecting the target cells detached from the antibody and the surface to form a population of target cells free of bound antibody.

In some embodiments, provided is a method for producing a bead-free population of target cells which comprises: a) contacting a mixed cell population with an antibody attached to a bead, the antibody specifically binds to the target cell and the antibody comprises an exogenous proteolytic cleavage site; b) separating the target cell bound to the bead from cells not bound to the beads; c) contacting the bead bound target cell with a protease, or portion of a protease, specific for the cleavage site to detach the target cell from the bead; and d) collecting the target cells detached from the beads to form a bead-free population of target cells.

The methods and compositions provided herein are for use in isolating or separating any type of cell from others in a mixed cell population so long as the target cell has an epitope that can be distinguished from the other cells in the population. In other embodiments, the methods and compositions are for use in isolating or separating any type of cell from non-cell components of a composition so long as the target cell has an epitope that can be distinguished from the unwanted components of the composition.

In some embodiments of the methods, at least about 15% of the target cells in the starting composition are recovered. In some embodiments, at least about 20%, at least about 25%, at least about 30%, at least about 35%, at least about 40%, or at least about 50% of the target cells are recovered. In certain embodiments, about 15%, 20%, 25%, 30%, 35%, 40%, 45%, 50%, 60%, 70%, 80%, or 90% of the target cells in the starting composition are recovered.

In some embodiments, viability of the isolated target cell population is greater than 50%. In other embodiments, viability of the isolated target cell population is greater than 60%, greater than 70%, greater than 80%, greater than 90%, or greater than 95%. In certain embodiments, viability of the isolated target cell population is about 50%, 60%, 70%, 80%, 90%, or 95%.

As described, the methods and compositions provided can be used for the enrichment of target cells in an isolated cell population. Accordingly, in some embodiments, purity of the isolated target cell population is greater than 50%, that is, the percentage of the total isolated cell population which are target cells is greater than 50%. In other embodiments, purity of the isolated target cell population is greater than 60%, greater than 70%, greater than 80%, greater than 90%, or greater than 95%. In certain embodiments, purity of the isolated target cell population is about 50%, 60%, 70%, 80%, 90%, or 95%.

To capture the target cell, a mixed cell population is incubated with the recombinant anti-target epitope antibody before, during, or after attachment of the recombinant antibody to the surface. In some embodiments, this incubation is performed with the cell and antibody suspension being agitated, for example without limitation, with a rolling motion, a rocking motion, or a stirring motion. In other embodiments, this incubation of the cell and antibody suspension is performed without agitation. This incubation occurs at a temperature and for a length of time suitable for the antibody to bind the epitope on the target cell. In some embodiments, the incubation occurs at room temperature. In other embodiments, the incubation occurs at 4°C, 8°C, 25°C, 30°C, or 37°C. In some embodiments, the incubation is for about 5 minutes to about 2 hours. In certain embodiments, without limitation, the incubation is for about 5 minutes, 10 minutes, 15 minutes, 20 minutes, 30 minutes, 40 minutes, 50 minutes, 60 minutes, 75 minutes, 90 minutes, or 105 minutes.

In provided methods, a target cell/recombinant antibody/surface complex is formed. The components of this complex can be brought together in several different ways. For example, in one embodiment, the composition comprising the target cell can be contacted with the anti-target epitope recombinant antibody attached to the surface. In another embodiment, the composition comprising the target cell can be contacted with the anti-target epitope recombinant antibody and after the target cell has bound the antibody, the antibody can be attached to the surface through, for example, another antibody which links the recombinant antibody to the surface. In yet another embodiment, the composition comprising the target cell can be simultaneously contacted with the anti-target epitope recombinant antibody and with the surface, the recombinant antibody having some means to link the recombinant antibody to the surface.

Thus, in another aspect, provided herein is a composition comprising a target cell, a recombinant antibody, and a solid surface, where the recombinant antibody is specifically bound to the cell and is also attached to the solid surface, and where the recombinant antibody comprises an exogenous proteolytic cleavage site.

The recombinant antibody can be directly attached to the surface or indirectly attached to the surface, for example, through the use of a linker, such as an antibody, which binds the recombinant antibody and which is attached to the surface.

In certain embodiments, the surface is a solid surface. Without limitation, the solid surface can be in the form of a cell culture well or culture plate, a particle or bead, a microscope slide, or a membrane. The solid surface can be magnetic or non-magnetic. In embodiments in which the surface is non-magnetic, separation of the target cells bound to the recombinant antibody/surface complex from non-target cells and material can be accomplished, for example, through washing, centrifugation, filtration and/or sieving. Non-magnetic particles suitable for use in the provided methods, such as polymer beads, are commercially available and techniques for attaching an antibody to such beads are well known in the art.

The use of magnetism in cell isolation techniques is well known and magnetic surfaces for use in such techniques are commercially available. Magnetic or magnetizable surfaces, such as particles, for use in the methods can be without limitation, magnetic, paramagnetic, or superparamagnetic. The term "magnetic" as used herein means that the support is capable of having a magnetic moment imparted to it when placed in a magnetic field, and thus is displaceable under the action of that field. Accordingly, magnetic particles may readily be removed from other components of a sample by magnetic aggregation, which provides a quick, simple, and efficient way of separating the particles following binding of the target cell and also following proteolytic release of the target cell from the antibody/particle complex. In addition, magnetic aggregation is a gentler separation method than techniques which generate shear forces, such as centrifugation.

Thus, magnetic particles may be removed or separated by application of a magnetic field, for example by using a permanent magnet. It is typically sufficient to apply a magnet to the side of the vessel containing the sample mixture to aggregate the particles to the wall of the vessel and to remove the remainder of the sample (e.g., the unbound, non-target cells) and/or the particles (e.g., following protease treatment). Alternatively, separation of the magnetic particles may be performed using an automated system for handling of such magnetic particles. Such automated systems and apparatuses are known and commercially available.

Application of a magnet can be for a time sufficient to segregate the magnetic particles from the remainder of the sample and will depend, for example, on the type of magnetic particle and the composition of the sample. Typically, paramagnetic or superparamagnetic particles of the micron and submicron sizes can be segregated to the side of a vessel containing an aqueous sample through application of a magnet for minutes, for example, 1 to 30 minutes. For example, particle segregation can occur through application of a magnet for 20 minutes or less. In some embodiments using magnetic particles as the as the surface to which the recombinant antibody is attached, segregation of the particles is accomplished through application of a magnet for 1 to 20 minutes. In some embodiments, application of a magnet is for 1, 2, 3, 4, 5, 6, 7, 8, 9, 10, 15, 20, or 30 minutes.

In some embodiments, magnetic particles for use in the cell separation methods are about 1-10 micrometers in diameter. In certain embodiments, the particle diameter is about 1 µm, 2 µm, 2.8 µm, 3.2 µm, 3.5 µm, 4.5 µm, 5 µm, 8 µm, or 10 µm. In other embodiments, the magnetic particles are less than 1 µm in diameter, for example, about 0.05 to 1 µm or about 0.09 to 0.6 µm. In certain embodiments, the particle diameter is about 50 nm, 90 nm, 135 nm, 175 nm, 200 nm, 250 nm, 300 nm, 400 nm, 500 nm, 600 nm, 700 nm, or 800 nm.

Magnetic particles suitable for use in the methods provided are of a variety of compositions including, but not limited to, fine grains of iron oxides dispersed throughout the interior of a polymer particle, silanized particles of magnetic iron oxides, and silanized particles of magnetic porous glass. Polymer compositions of magnetic polymer particles include, for example, polystyrene, cellulose, and latex. Commercially available magnetic particles are available with surfaces modified so as to allow for direct or indirect attachment of molecules, such as a capture antibody, to the particle surface. Such surface modifications of such particles include, but at not limited to, immobilized antibodies such as, without limitation, secondary antibodies, immobilized protein A, immobilized protein G, immobilized streptavidin, immobilized avidin, immobilized biotin, immobilized oligo (dT), and having end groups such as, without limitation, -COOH, -NH2, -OH, epoxy-, tosyl-activated, hydrazide, and glyceryl.

In some embodiments, the target cells are animal cells, such as mammalian cells, including but not limited to, human, non-human primate, murine, porcine, bovine, ovine, feline, canine, and rabbit cells. Target cells include, without limitation, blood cells, thymocytes, lymphocytes, bone marrow cells, splenocytes, umbilical cord blood cells, liver cells, corneal cells, epithelieal cells, endothelial cells, bone cells, adult stem cells, induced pluripotent cells, embryonic stem cells, and mesenchymal stem cells.

In certain embodiments, the target cells are T cells, including without limitation, helper T cells, cytotoxic T cells, memory T cells, regulatory T cells, natural killer T cells. In some embodiments, the target cells are CD3+ T cells. In some embodiments, the target cells are CD4+ T cells. In other embodiments, the target cells are CD8+ T cells. In still other embodiments, the target cells are CD 34+ cells or Tra-1-60 + cells.

In some embodiments, the mixed cell population from which the target cell is to be isolated is, without limitation, blood cells, such as peripheral blood mononuclear cells, liver cells, bone marrow cells, umbilical cord blood cells, splenocytes, endothelial cells, and epithelial cells.

In certain embodiments, the methods and compositions provided are for use in isolating a target cell based on an endogenously expressed epitope on the cell. In such methods, the recombinant antibody specifically binds an epitope endogenously expressed on the target cell.

In certain embodiments, the methods and compositions provided herein are for use in isolating a target cell which has been transfected or transduced to express an exogenous receptor or cell surface marker. In such methods, the recombinant antibody specifically binds an epitope of the exogenous receptor or cell surface marker on the target cell.

Target cells and the mixed cell populations may be primary cells, including without limitation, freshly isolated cells, cryopreserved cells, or cultured primary cells, or the cells may be immortalized cells, such as an immortalized cell line. In some embodiments, the primary or immortalized cell may be a transfected or transduced cell or cell line.

In one aspect, a recombinant antibody is provided which comprises an exogenous proteolytic cleavage site for a protease. The exogenous proteolytic site is a particular amino acid sequence which has been added to the antibody amino acid sequence and which can be cleaved by a particular protease. In some embodiments, the exogenous proteolytic cleavage site is inserted in the hinge region of the antibody. For example, the exogeneous cleavage site may be inserted in the upper hinge region, e.g., in the hinge region immediately following the CH1 domain, or may be inserted in the lower hinge region, e.g., in the hinge region immediately preceeding the CH2 domain.

The provided methods use a protease that specifically cleaves the exogenous proteolytic cleavage site inserted in the anti-target epitope antibody. In some embodiments, the protease is an endoprotease including, but not limited to, enteropeptidase, thrombin and Factor Xa. In some embodiments, the protease is not expressed in the target cell. In certain embodiments, the protease is a viral protease including, without limitation tobacco etch virus (TEV) protease, a rhinovirus 3C protease, a tobacco vein mottling virus (TVMV) protease, a plum pox virus protease, and a turnip mosaic virus protease. Other proteases with specific cleavage sites may also be used, as will be clear to the skilled artisan.

It is well known that proteases have catalytic domains and these can be used in place of full length proteases. Accordingly, the methods encompass the use of a full length protease, or an active portion thereof. It is also known that proteases may be modified to alter activity in useful ways, for example, increase activity, improve solubility, and/or decrease autolysis, and encompass the use of such modified proteases, or active portions thereof. Accordingly, in some embodiments, the protease is a TEV protease with an S219V modification, including the modified TEV protease AcTEV™ Protease (Invitrogen™, Life Technologies).

The reaction conditions for optimal antibody cleavage by the protease can vary depending, for example, on the cell type and the protease involved. For example, cleavage of the recombinant antibody by the protease may be improved by the presence of a reducing agent in the cleavage reaction solution. Accordingly, in some embodiments, a reducing agent is present when the target cell attached to the antibody-coated surface is contacted with the protease. In some embodiments, the presence of a reducing agent during the protease cleavage reaction increases the yield of detached target cells by greater than 50% as compared to the reaction in the absence of the reducing agent. In certain embodiments, the presence of a reducing agent during the protease cleavage reaction increases the yield of detached target cells by greater than 60%, greater than 70%, greater than 80%, or greater than 90% as compared to the reaction in the absence of the reducing agent.

Typically, a reducing agent that supports sufficient protease activity to cleave the antibody without significantly decreasing target cell viability is suitable for the methods provided. In some embodiments, a reducing agent is present during the protease cleavage reaction at a concentration which does not reduce cell viability by more than 50%. In certain embodiments, a reducing agent is present during the protease cleavage reaction at a concentration which does not reduce cell viability by more than 40%, more than 30%, more than 20%, or more than 10%. In other embodiments, a reducing agent is present during the protease cleavage reaction at a concentration that results in an isolated target cell population with greater than 50% cell viability. In certain embodiments, a reducing agent is present during the protease cleavage reaction at a concentration that results in an isolated target cell population with greater than 60%, more than 70%, more than 80%, or more than 90% cell viability.

Exemplary reducing agents include, but are not limited to TCEP (tris(2-carboxyethyl)phosphine), DTT (dithiothreitol), NAC (N-acetyl cholate), GSH (glutathione), thioglycolate and 2-ME (beta-mercaptoethanol). Such reducing agents may be used at concentrations including, but not limited to, 0.04 mM to 20 mM. In some instances, a reducing agent may be used at concentrations including, without limitation, 0.04 mM to 1 mM, 0.2 mM to 5 mM, 0.4 mM to 10 mM, or 0.8 mM to 20 mM. Exemplary temperatures for the protease reaction include, but are not limited to, 4oC, room temperature and 37oC. Exemplary incubation times for the protease cleavage step include, but are not limited to 15 minutes to 16 hours, such as 0.5 hour, 1 hour, 2 hours, 4 hours, 6 hours, 8 hours, 12 hours, or 16 hours. In some embodiments, the solution comprising the target cell/antibody/surface complex and protease is agitated during the protease cleavage reaction, for example without limitation, with a rolling motion, a rocking motion, or a stirring motion. In other embodiments, this reaction is performed without agitation.

In general, proteases that cleave a protein at a particular cleavage site are suitable for use in the provided methods. The particular proteolytic cleavage site is inserted into the recombinant antibody amino acid sequence through addition, deletion, and/or modification of amino acids to generate the exogenous proteolytic cleavage site. For many proteases, the amino acid requirements for the proteolytic cleavage sites are known. Exemplary proteolytic cleavage sites of the exemplary proteases for use in the methods include, but are not limited to, DDDDK (SEQ ID NO:1) for enteropeptidase (with cleavage occurring following the Lys), LVPRGS (SEQ ID NO:2) for thrombin (with cleavage occurring between the Arg and Gly), LVPRGS (SEQ ID NO:2) for Factor Xa (with cleavage occurring between the Arg and Gly), ENLYFQG (SEQ ID NO:3) for TEV protease (with cleavage occurring between the Glu and Gly), LEVLFQGP (SEQ ID NO:4) for rhinovirus 3C protease (with cleavage occurring between the Glu and Gly). Some amino acid variation at particular positions in the cleavage site sequence may be compatible with proteolytic cleavage by the protease while at other positions in the sequence, no variation is permitted. For example, a consensus TEV protease cleavage site has been defined as Glu-Xaa-Xaa-Tyr-Xaa-Gln-(Ser/Gly) (SEQ ID NO:5), with Xaa being any amino acid and with cleavage occurring between the Gln and Ser/Gly.

To generate the recombinant antibody, an exogenous proteolytic cleavage site is inserted at a position in the hinge region of the antibody through mutation or modification of amino acids in the antibody. Modification of the amino acid sequence of an antibody can be accomplished using methods well known in the art and commercially available reagents and kits.

The recombinant antibody may be generated using any antibody amino acid or nucleic acid sequence including, for example, mouse, human, rabbit, rat, chicken, goat, Camelid mammal (for example, llama, camel, and alpaca), and cartilaginous fish (for example, shark) antibody sequences. In some embodiments, the recombinant antibody may comprise portions or regions from more than one original antibody or source. For example, a recombinant antibody may contain constant regions and hinge region of a mouse antibody and variable regions of a rabbit antibody. Alternatively, a recombinant antibody may be entirely based on a mouse antibody sequences but the constant regions and variable region sequences may be from two different mouse antibodies. The recombinant antibody may comprise heavy chains from any of IgG, IgM, IgE, IgD, IgA, or IgY, or combinations thereof. The recombinant antibody may comprise either kappa or lamba light chains, or combinations thereof. The recombinant antibody may comprise only heavy chains and lack light chains, such as those found in Camelid mammals and cartilagenous fishes.

As described herein, the recombinant antibody may be a whole antibody molecule, a single chain antibody, or an antibody fragment or portion which retains the ability to exhibit epitope-binding activity. In addition to an epitope-binding portion, the recombinant antibody comprises an exogenous proteolytic cleavage site. Suitable antibody fragments include, but are not limited to, a Fab fragment, a single chain Fv fragment (where both light and heavy chains are present as a fusion protein), and a VHH fragment (a single heavy chain variable domain of an immunoglobulin naturally devoid of light chains, such as can be found, for example, in Camelid mammals and sharks).

The exogenous proteolytic cleavage site may be inserted into a generic constant region backbone of the antibody heavy chain. Variable regions with specificity for any specific cell surface marker can be combined in conjunction with the generic constant region backbone of a heavy chain containing the exogenous cleavage site. Generation of such recombinant antibodies can be accomplished by combining nucleic acid sequences encoding the variable regions of choice with nucleic acid sequences encoding the generic constant regions. Techniques and tools, such as reagents, cells, and devices, to create such recombinant antibodies are well known and commercially available to the skilled artisan.

Also provided are nucleic acid molecules which encode the recombinant antibody. For example, provided is a nucleic acid molecule encoding an antibody heavy chain comprising an exogenous proteolytic cleavage site. In some embodiments, an expression vector comprises the nucleic acid molecule encoding a heavy chain comprising an exogenous proteolytic cleavage site. Such an expression vector comprises a nucleic acid molecule encoding an entire heavy chain including the variable region and constant regions. Such an expression vector may comprise a nucleic acid molecule encoding the constant regions of the heavy chain with an insertion site for operatively introducing a nucleic acid molecule encoding a variable region of choice.

The following examples are provided by way of illustration and not by way of limitation.

### EXAMPLES

### Example 1 - Generation of expression constructs for encoding recombinant antibodies with an inserted proteolytic cleavage site

For the cloning of the heavy chain constant region (CH) gene of mouse IgG1, the mouse C gamma 1 gene was PCR amplified from genomic DNA isolated from hybridoma cells (BerEp4) using the primers; 5'mIgG1_HindIII gtaagctt tcg ggg aca tgg gaa tgc aaa agt agc (SEQ ID NO:6) and 3'mIgG1_BamHI gtggatcc ggg ctc aaa cca tgg aga ccc ct (SEQ ID NO:7) (restriction enzyme sites HindIII and BamHI are underlined). The blunt PCR product was TOPO cloned into linearized and topoisomerase I-activated pCR®-Blunt II-TOPO vector using Zero Blunt® TOPO® Cloning Kit (Invitrogen™, Life Technologies). A BsmI site in the intron upstream of CH1 of mouse IgG1 was removed by QuikChange® site-directed mutagenesis (Agilent Technologies) using primer 5' aaGCTTTCGGGGaCaTgGAAATGCAAAAGAGCGGCCttc (SEQ ID NO:8) (mutated BsmI site underlined).

The C gamma 1 gene was modified in the upper hinge region to insert the proteolytic cleavage site of TEV protease using QuikChange primers 5'mIgG1 TEV-Fab: 5'-cag Tg ccc agg gat gaa aac ctg tat ttt cag ggc tgt ggt tgt aag cc (SEQ ID NO:9) and a complementary 3' primer. The resulting amino acid sequence in the hinge region was PRDENLYFQGCGCKPCICT (SEQ ID NO:10) (the inserted TEV cleavage site underlined). The TEV cleavage site was introduced upstream of the cysteines in the hinge to enable cleavage into single Fab-fragments when subjected to a TEV protease, such as AcTEV™ protease (Invitrogen™, Life Technologies).

To clone the mouse IgG1-TEV construct into an expression vector with Variable (V-) and Constant (C)- region cloning cassettes, the constructed C gamma 1-TEV fragment was cut from the pCR®-Blunt II-TOPO vector using HindIII and EcoRI restriction enzymes and cloned into the antibody expression vector pLNOH2 (Norderhaug et al. (1997) J. Immunol. Methods 204(1):77-87) on respective restriction sites. pLNOH2 is based on pcDNA3.1, with immunoglobulin leader sequence and restriction sites for cloning any VH-genes and CH-genes. The resulting vector pLNO_mIgG1-TEV contains a V-region cassette (BsiWI/BsmI) for cloning of any VH-region in combination with the mouse C gamma 1-TEV.

For the cloning of the light chain constant region gene of mouse kappa, the mouse C kappa (mCk) was PCR amplified from genomic DNA isolated from hybridoma cells (BerEp4) using the primers 5' mCk HindIII: 5'-ttat aagctt cttaccttatgtgcttgtg (SEQ ID NO:11) and 3' mCk BamHI: 5'- atat ggatcc tttgtct cta aca ctc att cc (SEQ ID NO:12) (restriction enzyme sites HindIII and BamHI are underlined). The blunt PCR product was TOPO cloned into linearized and topoisomerase I-activated pCR®-Blunt II-TOPO vector using Zero Blunt® TOPO® Cloning Kit (Invitrogen™, Life Technologies).

To clone the nucleic acid molecule encoding mouse C kappa into an expression vector, the cloned mCk fragment was cut from pCR®-Blunt II-TOPO vector using HindIII and BamHI and cloned into pLNOk. The resulting vector pLNO_mCk contains a VL-region cassette (BsiWI/BsmI) for cloning of any VL-region in combination with the mouse C kappa region.

### Example 2 - Cloning Variable Heavy (VH) and Variable Light (VL) Chain regions genes from anti-human CD3 expressing hybridoma cell line into expression vectors

VH and VL chain genes were PCR amplified from poly dCTP 3'-tailed cDNA. The cDNA was synthesized from total RNA isolated from SPV-3tB hybridoma cells expressing anti-human CD3 antibodies using a gene specific 3' primer (cDNA kappa: tgc tgt ctt tgc tgt cct gat (SEQ ID NO: 13) and cDNA_IgG2a; tag agg tca gac tgc agg aca (SEQ ID NO: 14)). The V-genes were PCR amplified from the poly dCTP-modified cDNA using a 5' poly G primer and a C-region gene specific 3' primer (3' IgkC_rev: caa gaa gca cac gac tga ggc (SEQ ID NO:15), and 3' IgG2a_rev: ctt gac cag gca tcc tag agt ca (SEQ ID NO: 16)). The blunt PCR products were TOPO cloned into linearized and topoisomerase I-activated pCR®-Blunt II-TOPO vector using Zero Blunt® TOPO® Cloning Kit, sequenced and analysed for productive V-gene sequence (IMGT database).

New V-region specific primers, with BsmI and BsiWI restriction enzyme sites to enable cloning into the backbone expression vectors pLNO_mIgG1-TEV and pLNO_mCK (Example 1), were synthezized to PCR amplify the cloned VH and VL sequences from the pCR®-Blunt II-TOPO vectors. Primers for anti-human CD3 VL were 5' VL_BsmI: 5'-attc tgcattc caa att gtt ctc acc cag tct cca gc (SEQ ID NO: 17) and 3' VL_BsiWI: 5'-tcga cgtacg ttct **actc acg t**tt cag ctc cag ctt ggt cc (SEQ ID NO: 18). Primers for anti-human CD3 VH were 5' VH_BsmI: 5'-tc tgcattc cag gtc cag ctg cag cag (SEQ ID NO:19) and 3' VH_BsiWI: 5'-ga cgtacg ttct **actc acc t**ga gga gac ggt gac tga cc (SEQ ID NO:20) (restriction enzyme site underlined and splice site in bold). The PCR products encoding VH and VL chain genes were restriction enzyme cut using BsmI and BsIWI and ligated into their corresponding backbone vector described above, resulting in a complete genomic L-chain gene expression vector (pLNO_mCk-SPV) and a complete genomic H-chain gene expression vector (pLNO_mIgG1TEV-SPV) encoding recombinant antibodies with specificity for human CD3 and with the TEV proteolytic cleavage site genetically inserted the upper hinge region.

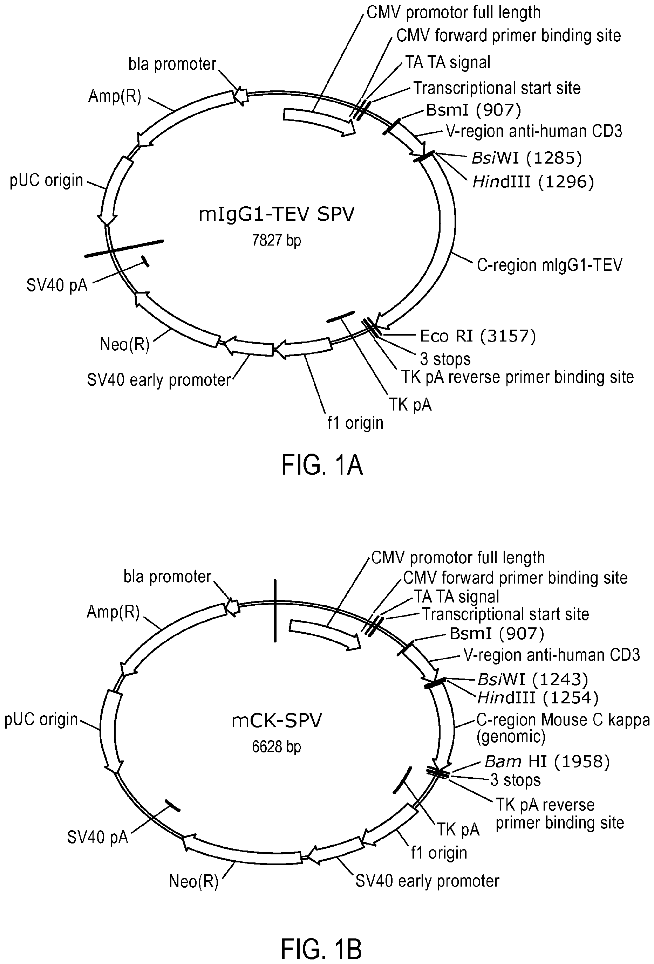

The complete V- and C-region cassettes including the leader region from the vectors pLNO_mCk-SPV and pLNO_mIgG1TEV-SPV were PCR amplified and TOPO cloned into a pcDNA3.3 vector with a mutated BsmI site in the SV40 polyA site. The 5' primer upstream of the leader sequence was used for both the H-, and L-chain vector (5' Leader: 5'- cca agctagc ttggtaccg (SEQ ID NO:21)). The 3' primer was constructed specifically for mCk (3' mCk: 5'- ggatcctttgtctctaacac (SEQ ID NO:22)) or mIgG1-TEV (3'm IgG1: 5'-gaattcgcccttgtggatc (SEQ ID NO:23)). The PCR products including the leader sequence and Kozak site were amplified using Taq polymerase to ensure TA' overhangs necessary for TOPO cloning using pcDNA3.3-TOPO® Cloning kit (Invitrogen) and correct orientation of the insert downstream of the CMV promotor was verified. Maps of the H-chain and L-chain expression vectors are depicted in Fig. 1 (mIgG1-TEV SPV (Fig. 1A) and mCk-SPV (Fig. 1B)).

### Example 3 - Expression of recombinant antibody with an inserted proteolytic cleavage site

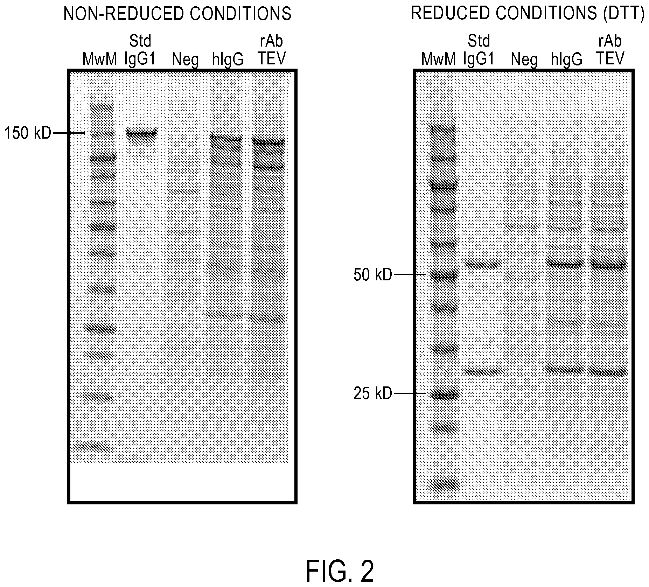

Vectors encoding the anti-CD3 L-chain and H-chain were co-transfected into suspension FreeStyle™ 293-F cells (Invitrogen) using FreeStyle™ 293F Expression System in ratio 1:2 heavy and light chain. The supernant was harvested at day 5 and analysed on a Coomassie gel (NuPAGE® gel, Novex®, Life Technologies) under reduced (with DTT) and non-reduced conditions (Fig. 2). Recombinant antibody expression was estimated to vary between 40-80 µg/ml. The recombinant antibodies were purified from supernantant using Dynabeads® Protein G (Invitrogen™, Life Technologies) and eluted in 50 mM Glycine pH 2.8. The eluate was neutralized to pH 7 by adding Tris-HCl pH 8. The recombinant anti-human CD3 antibody with TEV cleavage site in the upper hinge region of mouse IgG1 is herein referred to as rAb-TEV.

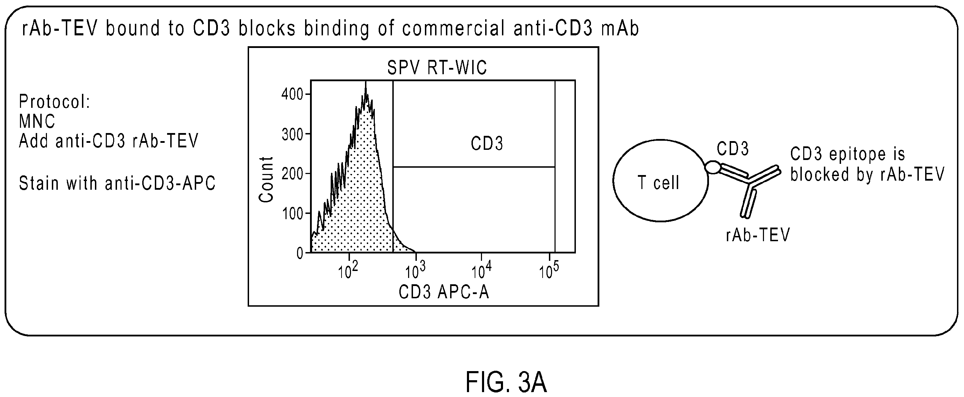

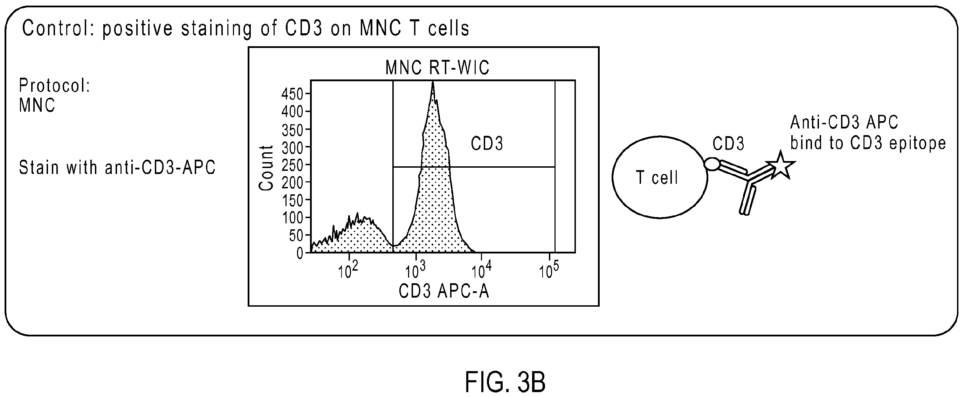

To demonstrate V-region specificity of the constructed rAb-TEV, the purified rAb-TEV was added to human mononuclear cells (MNC) prior to staining with commercially available anti-human CD3 antibody conjugated with APC (anti-CD3-APC). MNC were also stained with anti-CD3-APC alone as a positive control. The cells were then analyzed by flow cytometry. The rAb-TEV blocked staining by the anti-human CD3 antibody, indicating that rAb-TEV binds to the same CD3 epitope (Fig. 3 A-B). This result demonstrates that the cloned VH-and VL-chain regions maintained their specificity for human CD3.

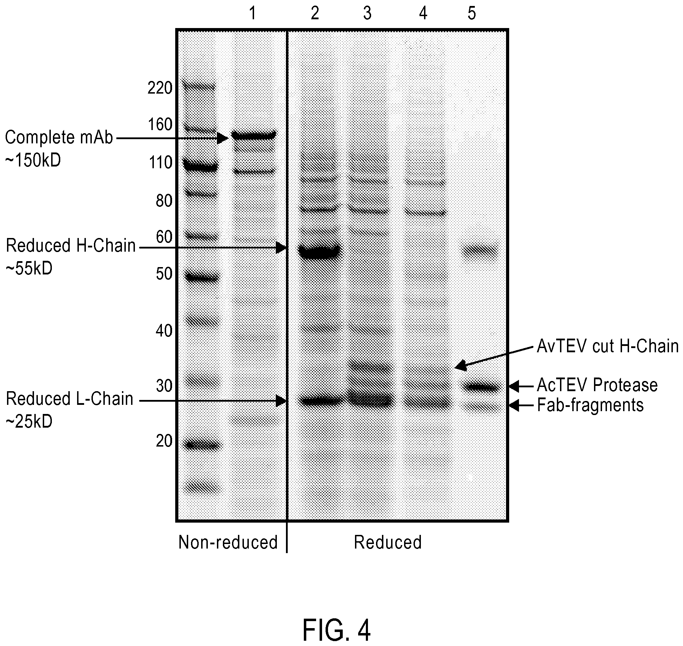

Purified rAb-TEV was incubated with AcTEV™ Protease (Invitrogen™, Life Technologies) in enzyme buffer (1 mM DTT) and the cleaved protein fragments were analyzed on SDS-PAGE Coomassie blue gel under reduced and non-reduced conditions. The results are shown in Fig. 4. When treated with AcTEV™ Protease, the complete IgG1 was cleaved into Fc-fragments and single Fab-fragments demonstrating that the cleavage site is accessible to the protease in the recombinant antibody when inserted in the upper hinge region.

### Example 4 - Recombinant antibody attached to solid surface

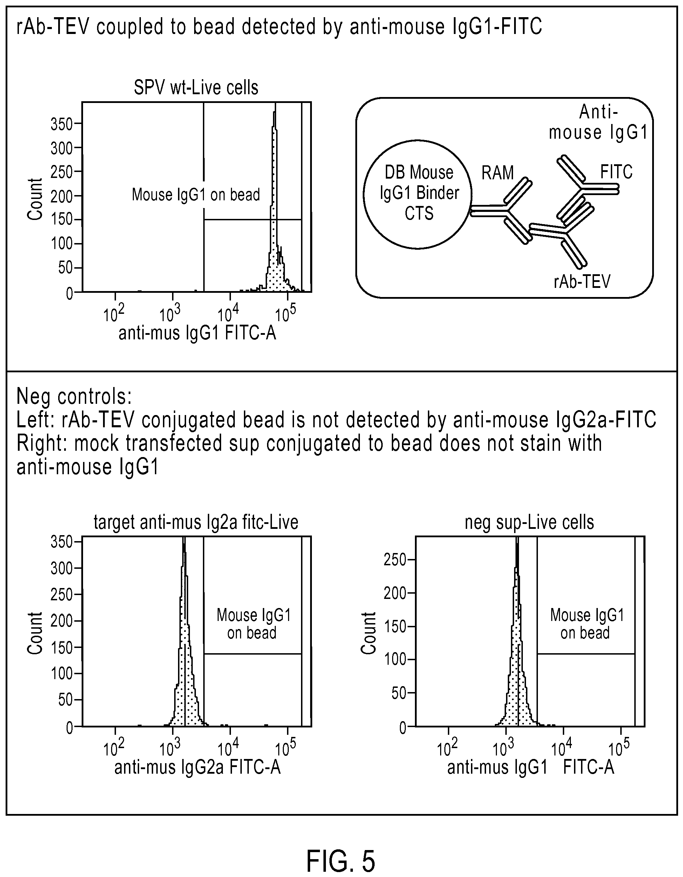

Purified rAb-TEV was coupled to Dynabeads® Mouse IgG1 Binder (Invitrogen™, Life Technologies) which are 4.5 µm paramagnetic particles conjugated with a monoclonal rat-anti mouse IgG1 antibody (RAM). The coupled rAb-TEV of IgG1 isotype could be detected in flow by FITC conjugated anti-mouse IgG1 Ab (Fig. 5, top panel). Negative controls used were FITC conjugated anti-mouse IgG2a (Fig. 5, bottom left) and mock transfected supernanant bound to beads (Fig. 5, bottom right) which were not detected by FITC conjugated anti-mouse IgG1 Ab. These results demonstrate specific binding of mouse IgG1 rAb-TEV to Dynabeads® Mouse IgG1 Binder.

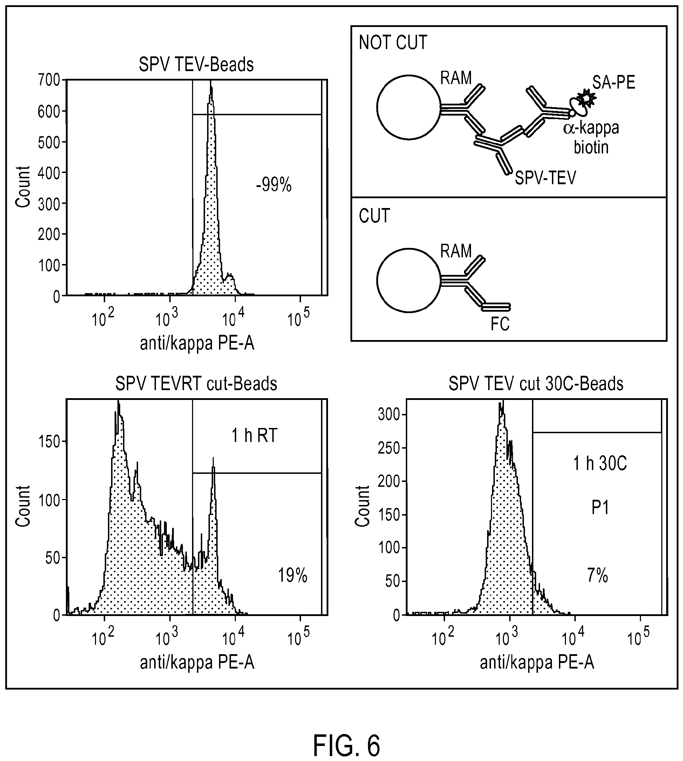

To assess cleavage of rAb-TEV immobilized to a solid surface, rAb-TEV coupled to Dynabeads® Mouse IgG1 Binder beads were incubated with AcTEV™ Protease for 1 hour at room temperature or 37°C. Without AcTEV™ Protease, the beads stained brightly by a PE-conjugated anti-kappa Ab in flow cytometry analysis with 99% of the beads kappa-positive (Fig. 6, top left). However, after a one hour incubation with the protease, the kappa-positive faction of beads was greatly reduced to 19% for room temperature incubation (Fig. 6, bottom left) and to 7% for 37°C incubation (Fig. 6, bottom right), indicating that the Fab-fragments had been cleaved from the Fc-region of rAb-TEV/bead complex. A cartoon depicting the two scenarios is shown in Fig. 6, top right. Accordingly, the inserted exogenous proteolytic cleavage site was accessible to the protease when the recombinant antibody was attached to the solid surface.

### Example 5 - Depletion of target cells from mixed population using recombinant antibody attached to solid surface

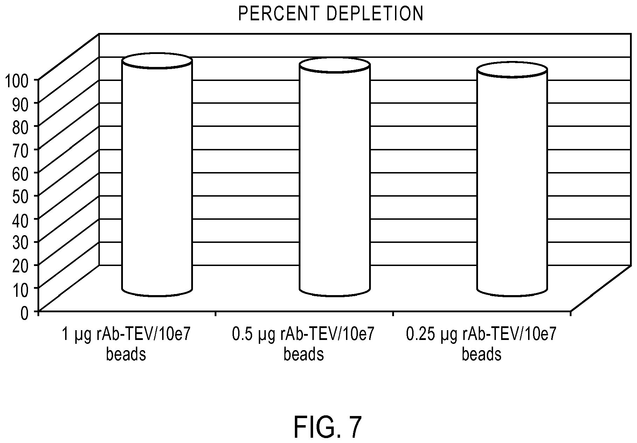

The rAb-TEV was coupled to Dynabeads® Mouse IgG Binder at different concentrations and used to deplete CD3⁺ T cells from a mixed population of mononuclear cells (MNC). Four beads per CD3⁺ target cell were added and percentage of depleted CD3⁺ T cell was analysed by flow cytometry after 20 minutes incubation. All beads efficiently depleted CD3+ T cells in the MNC population with all of the 3 different rAb-TEV/Dynabeads tested binding and depleting more than 94% of CD3⁺ T cells (range 94-98%, Fig. 7). The depletion efficiency is similar to Dynabeads CD3 which is the corresponding 4.5 µm magnetic particle covalently conjugated with the SPV-3tB anti-human CD3 Ab (the parent monoclonal Ab of rAb-TEV from where the anti-human CD3 V-regions were cloned), indicating that the V-region genes cloned into rAb-TEV maintained their specificity and functionality when bound to Dynabeads® Mouse IgG1 Binder. The depletion was robust in the range tested (0.25 to 1 µg rAb-TEV per 10E7 beads).

### Example 6 - Isolation of bead-free CD3+ T cells using recombinant antibody attached to beads plus protease

To obtain cell release after bead capture, the protease must have access to the proteolytic cleavage site which could be hidden in the close bead-cell complex. Also, to release a cell from the bead, the majority of epitope bound recombinant antibody (e.g., CD3 bound rAb-TEV) must be cleaved. The density of recombinant antibody coupled to the beads could therefore be a factor in achieving an efficient release and high recovery of isolated cells. Using the anti-CD3 recombinant antibody rAb-TEV and Dynabeads® Mouse IgG Binder, 4 different bead complexes with different amounts of rAb-TEV bound were tested.

Peripheral MNC were isolated from a healthy donor. Four rAb-TEV coupled Dynabeads were added per target CD3+ T cell in the mixed cell sample. The cells were incubated on a roller for 20 min before the non-bead bound cells were removed by placing the tube in a magnet. The washed bead-cell complexes were resuspended in 400 µl release buffer containing 10 U AcTEV™ Protease and incubated on a roller for 2 hours at room temperature. After incubation, the sample was mixed well and placed in the magnet to remove the beads. The supernatant containing the isolated bead-free cells was transferred to a new tube where recovery and purity of the isolated sample was analyzed. A cartoon of the workflow is depicted in Fig. 8.

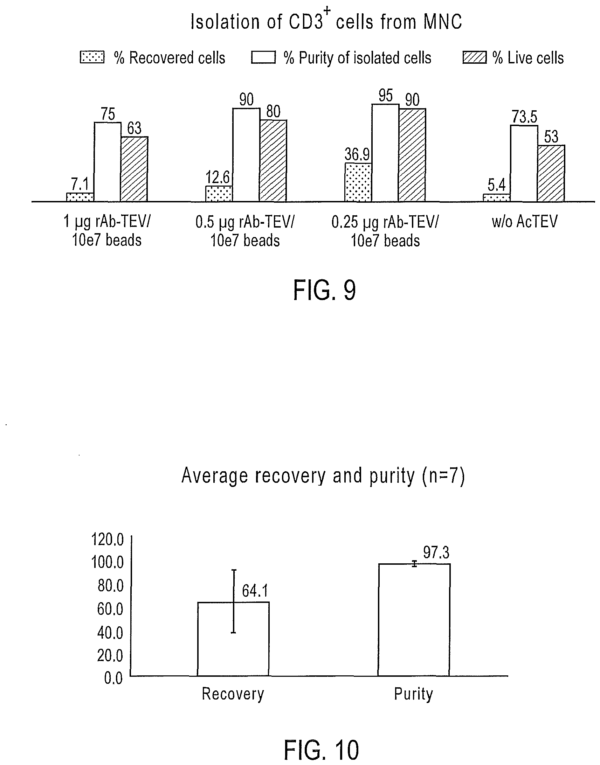

The isolated cell samples were analysed by flow cytometry and the resulting yield, purity and viability are shown in Fig. 9. With a low density of rAb-TEV bound to the beads (0.25 µg per 10E7 beads), 37% of target cells were recovered. With a density of rAb-TEV bound to the beads of 0.5 µg per 10E7 beads, 12.6% of target cells were recovered. In contrast, using 1 µg rAb-TEV bound per 10E7 beads, only 7% of target cells were recovered in the isolated cells sample which was similar to the control where no enzyme was added (5%). In this experiment, the highest recovery of target CD3+ T cells was observed when using the preparation with 0.25 µg rAb-TEV/10E7 Dynabeads Mouse IgG1 Binder. The purity and the viability of these isolated cells were also high (95% and 90%, respectively).

Peripheral MNCs from healthy donors were incubated for 20 min at RT with Dynabeads® Mouse IgG1 Binder coupled with 0.25 µg rAB-TEV per 10E7 beads for cell capture. The cells bound to the rAB were separated from unbound cells using a magnet. The washed bead-cell complexes were resuspended in release buffer containing 10 U AcTEV™ Protease and incubated for 2 hours at room temperature. Beads were removed by magnet and the bead-free, positively isolated CD3+ T cells recovered. After performing this isolation procedure with MNCs from seven healthy donors (n=7), the average recovery of target cells was 64.1% (STDEV 27) and average purity was 97.3% (STDEV 2.2) (Fig. 10). Under these conditions, cell viability was >90%, cell expression of CD2, CD3, CD4, and CD8 was unaffected, and the remaining rAB-TEV Fab fragments were not detected on the isolated cells.

### Example 7 - Isolated cells are bead-free and recombinant antibody-free

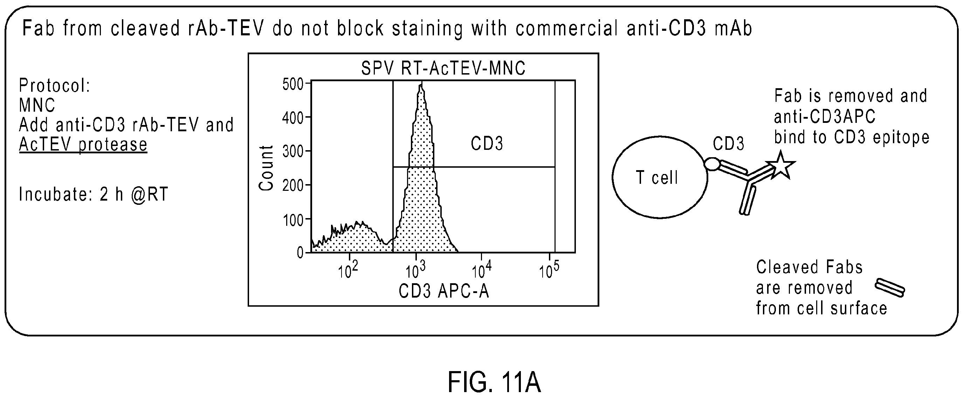

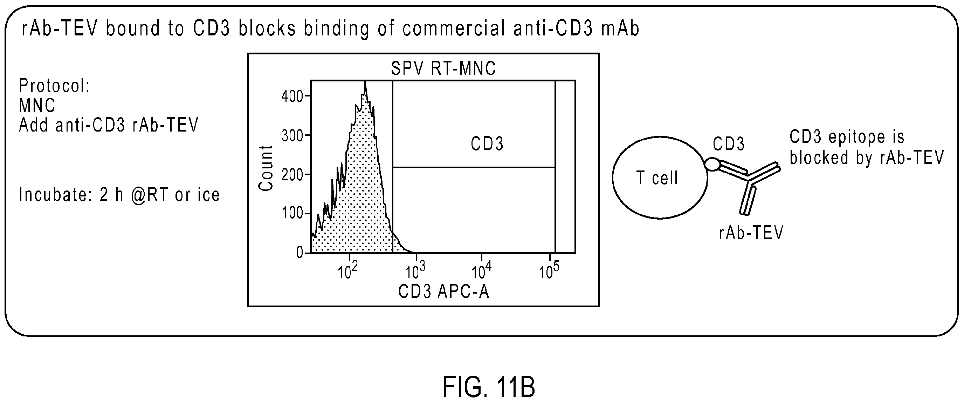

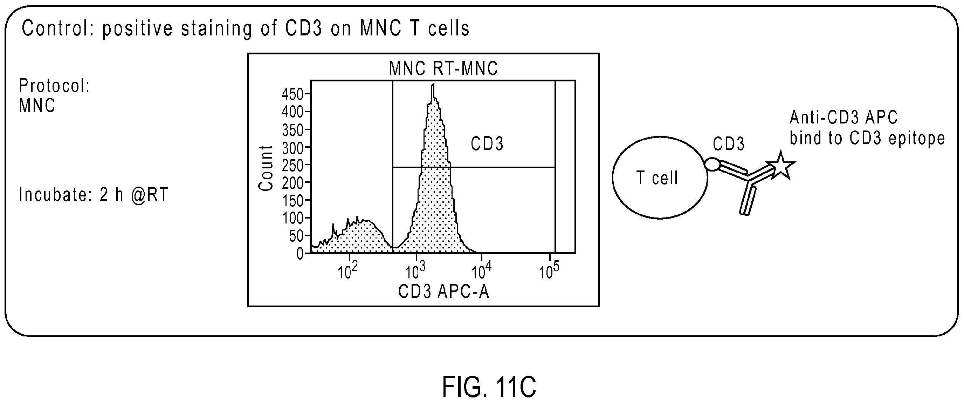

Following cell capture with the recombinant antibody coupled beads, cell release from the beads using AcTEV™ Protease cleaves the CD3 bound rAb-TEV in the upper hinge region resulting in Fab fragments from rAb-TEV that could remain bound to the CD3 epitope on the isolated cells. However, as Fab-fragments have reduced avidity compared to complete IgG antibody, CD3 bound Fab fragments may have higher turn off rate than complete IgG. To assess this, a commercially available anti-human CD3 antibody conjugated with APC (anti-CD3-APC) was used to stain MNC that had been incubated with rAb-TEV with or without the AcTEV™ Protease present. As shown in Fig. 11, the data show that when the protease was present, the CD3 epitope on the cell was no longer blocked by the rAb-TEV and the cells stained with the anti-CD3-APC (Fig. 11A), indicating that Fabs have been effectively removed from the cells after isolation. When the protease was not present, the CD3 epitope on the cell was blocked by the rAb-TEV and the cells did not stain with the anti-CD3-APC (Fig. 11B). Control cells incubated without rAb-TEV and AcTEV™ Protease stained with the anti-CD3-APC (Fig. 11C). Staining the cell populations with labeled anti-kappa antibody support the results found with anti-CD3-APC. These data indicate that the rAb-TEV Fab fragments were effectively removed from the CD3 epitope after cell isolation leaving bead-free and antibody-free cells.

### Example 8 - Activation and expansion of positively isolated CD3+ T cells

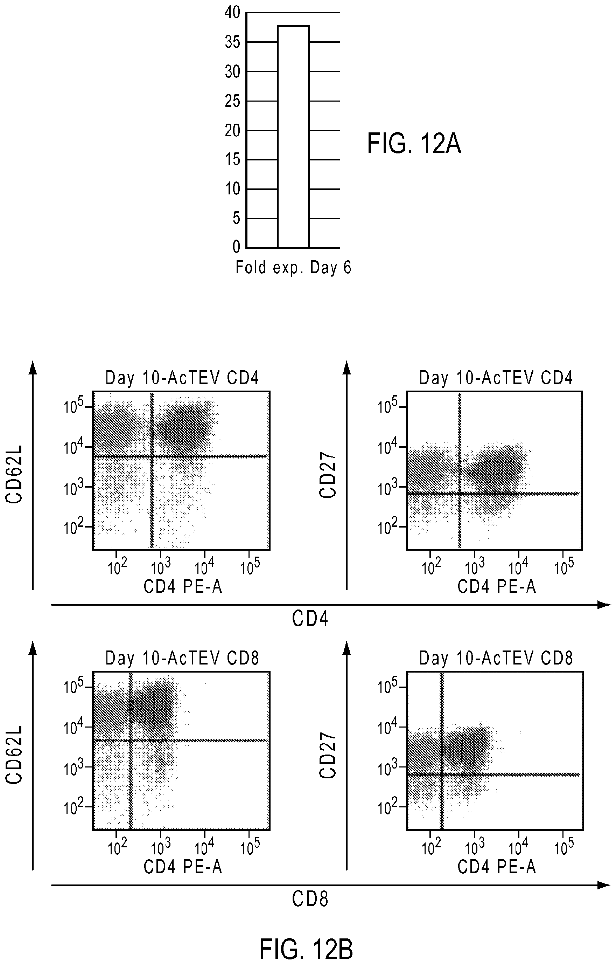

Recombinant antibody rAb-TEV bound to Dynabeads and AcTEV™ Protease were used as described in Example 6 to positively isolate CD3+ T cells from MNCs. These isolated T cells were then activated and expanded in culture using Dynabeads® CD3/CD28 CTS™ according to manufacturer's protocol (Gibco®, Life Technologies). The positively isolated T cells were activated and expanded, showing more than 35 fold expansion by day 6 (Fig. 12A) and a central memory phenotype as shown by CD62L and CD27 expression (Fig. 12B). This amount of activation and expansion is as expected for this type of cell and shows that the isolated cells behave normally. Thus, the cells isolated using the rAb-TEV/bead complex followed by TEV protease treatment were able to be activated and expanded as would be expected for CD3+ T cells isolated from MNC.

### Example 9 - Anti-human CD34 recombinant antibodies with protease cleavage site

The variable heavy chain region and variable light chain region from the anti-human CD34 hybridoma 561 were cloned into the expression vectors mIgG1-TEV and mCk, respectively, as described above for the anti-CD3 recombinant antibodies. The recombinant antibodies were expressed in FreeStyle™ 293F cells, purified using Protein G, and conjugated to Dynabeads® Mouse IgG1 as described for rAb-TEV with specificity for CD3. See Examples 2-4, for example.

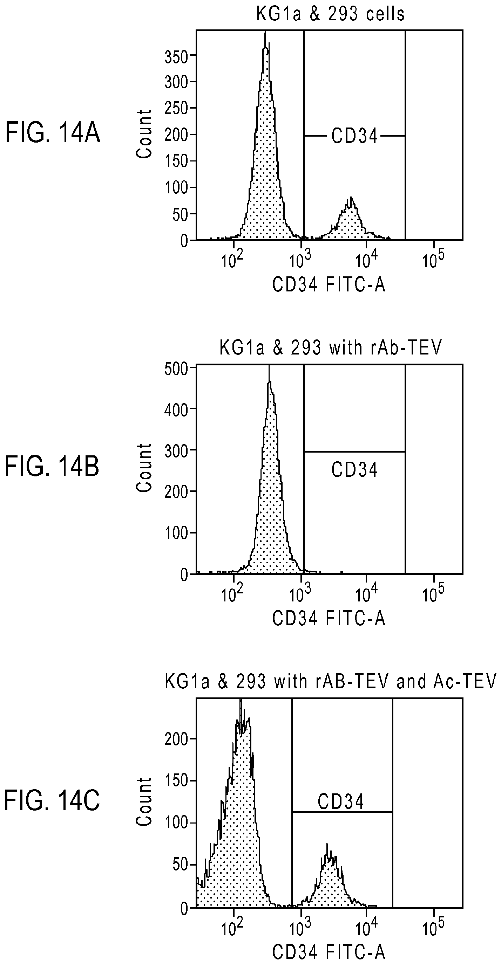

To demonstrate V-region specificity of the constructed rAb-TEV with specificity for CD34 (herein referred to as "rAb-TEV anti-CD34"), the purified rAb-TEV was added to mix of human cells: CD34+ KG1a cells and CD34- FreeStyle™ 293F cells prior to staining with commercially available anti-human CD34 antibody conjugated with FITC (anti-CD34-FITC). The cell mix was also stained with anti-CD34-FITC alone as a positive control. The cells were then analyzed by flow cytometry. The rAb-TEV anti-CD34 blocked staining by the anti-human CD34 antibody, indicating that rAb-TEV binds to the same CD34 epitope (Fig. 14, A and B). This result demonstrates that the cloned VH-and VL-chain regions maintained their specificity for human CD34.

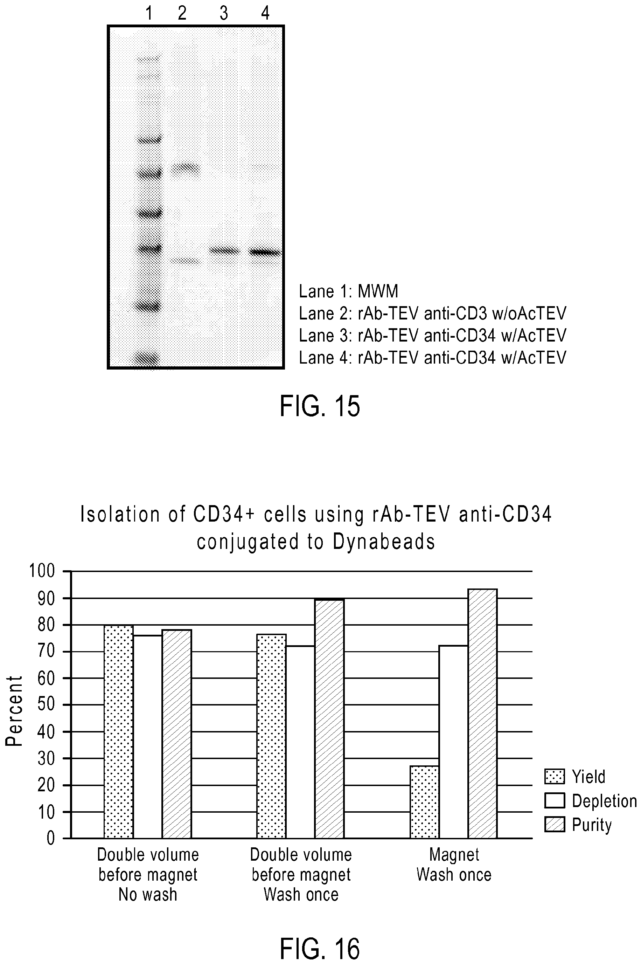

Purified rAb-TEV CD34 was incubated with or without AcTEV™ Protease for 2 hours at room temperature (22°C) and then analyzed on an SDS-PAGE Coomassie blue gel under reduced conditions. The results are shown in Fig. 15. When treated with the protease, the complete IgG1 was cleaved into Fc-fragments and single Fab-fragments demonstrating that the cleavage site is accessible to the protease in the recombinant antibody.

### Example 10 - rAb-TEVAnti-CD34 and isolation of CD34+ target cells

rAb-TEV anti-CD34 conjugated to Dynabeads® mouse IgG1 was used to positively isolate CD34⁺ KG1a cells as target cells from a mixed cells population of KG1a cells and FreeStyle™ 293F cells. The density of rAb-TEV anti-CD34 bound to the beads was 0.25 µg per 10E7 beads and four rAb-TEV anti-CD34 coupled Dynabeads were added per target CD34⁺ KG1a cell in the mixed cell sample. The cells were incubated on a roller for 20 minutes before the non-bead bound cells were removed by placing the tube in a magnet.

Samples of cells were subjected to varying wash conditions prior to being subject to identical enzymatic cleavage conditions. In one sample (Fig. 16, "double volume before magnet"), a volume of PBS equal to the volume in the tube containing the beads and cells was added to the tube, the tube placed in the magnet, and the non-bead bound cells removed. In another sample (Fig. 16, "double volume before magnet; wash once"), a volume of PBS equal to the volume in the tube containing the beads and cells was added to the tube, the tube placed in the magnet, and the non-bead bound cells removed. An additional volume of PBS wash was added to the bead-bound cells, the tube placed in the magnet, and the non-bead bound cells removed. In another sample (Fig. 16, "magnet; wash once"), the tube containing the beads and cells was placed directly in the magnet (without adding PBS) and the non-bead bound cells removed. A volume of PBS was added to the bead-bound cells, the tube placed in the magnet, and the non-bead bound cells removed.

The washed bead-cell complexes were resuspended in 400 µl per 10 million cells of release buffer containing 10 U AcTEV™ Protease and incubated on a roller for 2 hours at room temperature. After incubation, the sample was mixed well and placed in the magnet to remove the beads. The supernatant containing the isolated bead-free cells was transferred to a new tube and the cell samples were analyzed by flow cytometry for the resultant recovery, depletion, and purity. Results of exemplary CD34+ cell isolations are shown in Fig. 16.

The beads efficiently depleted CD34+ KG1a cells from the mixed population of cells. Target cells were efficiently recovered and the purity of the isolated cells was also high. It was generally found that bead-free CD34+ cells were isolated with comparable purity and recovery as was obtained for the CD3+ T cells described herein.

To assess whether Fab fragments from rAb-TEV anti-CD34 were present on the surface of isolated, bead-free cells following protease treatment, KG1a cells were incubated with rAb-TEV anti-CD34 with or without the AcTEV™ Protease present for 2 hours prior to washings and staining with a commercially available anti-CD34 antibody. Anti-CD34-FITC was then used to stain KG1a cells that had been incubated with rAb-TEV with or without the protease.

As shown in Fig. 14 B and C, the when the protease was present, the CD34 epitope on the cell was no longer blocked by the rAb-TEV anti-CD34 and the cells stained with the anti-CD34-FITC (Fig. 14C), indicating that CD34-bound Fab fragments have been effectively removed from the cells by the protease. These data indicate that this isolation method results in bead-free and antibody-free cells.

## Claims

1. A method for detaching a target cell attached to a recombinant antibody-coated solid surface, comprising: contacting the target cell attached to the surface with a protease, or portion of a protease, specific for an exogenous proteolytic cleavage site in the antibody, thereby detaching the target cell from the surface, wherein the recombinant antibody comprises an exogenous proteolytic cleavage site for a protease selected from the group consisting of a tobacco etch virus (TEV) protease, a rhinovirus 3C protease, a TVMV protease, a plum pox virus protease, a turnip mosaic virus protease, enteropeptidase, thrombin and Factor Xa, and wherein the exogenous proteolytic cleavage site is in the hinge region.

2. A method for isolating a target cell from a mixed cell population, comprising:
a) contacting a mixed cell population with an antibody attached to a solid surface, wherein the antibody specifically binds to the target cell in the population and wherein the antibody comprises an exogenous proteolytic cleavage site for a protease selected from the group consisting of a tobacco etch virus (TEV) protease, a rhinovirus 3C protease, a TVMV protease, a plum pox virus protease, a turnip mosaic virus protease, enteropeptidase, thrombin and Factor Xa, and wherein the exogenous proteolytic cleavage site is in the hinge region.

3. The method of claim 2, further comprising:
b) separating the target cells bound to the surface from cells not bound to the surface;
optionally further comprising c) contacting the target cells bound to the surface with a protease, or portion of a protease, specific for the cleavage site to detach the target cells from the surface; and optionally further comprising d) collecting the target cells detached from the surface.

4. A method for producing a population of activated target cells, comprising:
a) contacting a cell population comprising target cells with an agonistic antibody attached to a solid surface, wherein the antibody specifically binds to the target cells in the population and wherein the antibody comprises an exogenous proteolytic cleavage site for a protease selected from the group consisting of a tobacco etch virus (TEV) protease, a rhinovirus 3C protease, a TVMV protease, a plum pox virus protease, a turnip mosaic virus protease, enteropeptidase, thrombin and Factor Xa, and wherein the exogenous proteolytic cleavage site is in the hinge region; and
b) incubating the cells and the antibody attached to the surface under conditions to allow activation of the target cells.

5. The method of claim 4, further comprising:
c) separating the target cells bound to the surface from cells not bound to the surface;
optionally further comprising d) contacting the target cells bound to the surface with a protease, or portion of a protease, specific for the cleavage site to detach the target cells from the surface; and optionally further comprising e) collecting the activated target cells detached from the surface.

6. The method of claim 3, wherein the solid surface is a bead comprising:
a) contacting a mixed cell population with an antibody attached to beads, wherein the antibody specifically binds to the target cell in the population and wherein the antibody comprises an exogenous protease cleavage site for a protease selected from the group consisting of a tobacco etch virus (TEV) protease, a rhinovirus 3C protease, a TVMV protease, a plum pox virus protease, a turnip mosaic virus protease, enteropeptidase, thrombin and Factor Xa, and wherein the exogenous proteolytic cleavage site is in the hinge region;
b) separating the target cells bound to the beads from cells not bound to the beads;
c) contacting the target cells bound to the beads with a protease, or portion of a protease, specific for the cleavage site to detach the target cells from the beads; and
d) collecting the target cells detached from the beads to form a bead-free population of target cells.

7. The method of any one of claims 1, 3, 5 and 6, wherein the protease is not expressed by the target cell.

8. The method of any one of claims 1-6, wherein the cleavage site is in the upper hinge region of the antibody.

9. The method of any one of claims 2-6, wherein the cell population comprises stem cells or comprises blood cells, optionally wherein the blood cells are peripheral blood mononuclear cells.

10. The method of any one of claims 1-6, wherein the target cell is a T cell, a stem cell or wherein the target cell is CD34+.

11. The method of any one of claims 1-6, wherein the antibody specifically binds to an endogenous protein on the target cell surface or to an exogenous protein on the target cell surface.

12. The method of claim 11, wherein the antibody specifically binds a CD3 epitope, a Tra-1-60 epitope or a CD34 epitope.

13. The method of any one of claims 1-6, wherein the antibody is indirectly attached to the solid surface, preferably wherein the solid surface is a bead, optionally wherein the bead is paramagnetic.

14. The method of claim 4, wherein the target cell is a T cell and the antibody is an anti-CD3 antibody and optionally, wherein the incubating of (b) further comprises IL-2.

## Patentansprüche

1. Verfahren zum Ablösen einer Zielzelle, die an einer rekombinanten, mit Antikörpern beschichteten festen Oberfläche befestigt ist, umfassend: Kontaktieren der an die Oberfläche gebundenen Zielzelle mit einer Protease oder einem Teil einer Protease, die für eine exogene proteolytische Spaltstelle im Antikörper spezifisch ist, wodurch die Zielzelle von der Oberfläche abgetrennt wird, wobei der rekombinante Antikörper eine exogene proteolytische Spaltstelle für eine Protease umfasst, ausgewählt aus der Gruppe bestehend aus einer Tabakätzvirus-(TEV)-Protease, einer Rhinovirus-3C-Protease, einer TVMV-Protease, einer Pflaumenpockenvirusprotease, einer Rübenmosaikvirusprotease, Enteropeptidase, Thrombin und Faktor Xa, und wobei die exogene proteolytische Spaltstelle im Scharnierbereich liegt.

2. Verfahren zur Isolierung einer Zielzelle aus einer Mischzellpopulation, umfassend:
a) Kontaktieren einer Mischzellpopulation mit einem an eine feste Oberfläche gebundenen Antikörper, wobei der Antikörper spezifisch an die Zielzelle in der Population bindet und wobei der Antikörper eine exogene proteolytische Spaltstelle für eine Protease umfasst, ausgewählt aus der Gruppe bestehend aus einer Tabakätzvirus-(TEV)-Protease, einer Rhinovirus-3C-Protease, einer TVMV-Protease, einer Pflaumenpockenvirusprotease, einer Rübenmosaikvirusprotease, Enteropeptidase, Thrombin und Faktor Xa, und wobei die exogene proteolytische Spaltstelle im Scharnierbereich liegt.

3. Verfahren nach Anspruch 2, ferner umfassend:
b) Trennen der an die Oberfläche gebundenen Zielzellen von Zellen, die nicht an die Oberfläche gebunden sind;
optional ferner umfassend c) Kontaktieren der an die Oberfläche gebundenen Zielzellen mit einer Protease oder einem Teil einer Protease, die für die Spaltstelle spezifisch ist, um die Zielzellen von der Oberfläche zu lösen; und
optional ferner umfassend d) Sammeln der von der Oberfläche abgelösten Zielzellen.

4. Verfahren zur Herstellung einer Population von aktivierten Zielzellen, umfassend:
a) Kontaktieren einer Zellpopulation, die Zielzellen umfasst, mit einem agonistischen Antikörper, der an eine feste Oberfläche gebunden ist, wobei der Antikörper spezifisch an die Zielzellen in der Population bindet und wobei der Antikörper eine exogene proteolytische Spaltstelle für eine Protease umfasst, die ausgewählt ist aus der Gruppe bestehend aus einer Tabakätzvirus (TEV)-Protease, einer Rhinovirus-3C-Protease, einer TVMV-Protease, einer Pflaumenpockenvirusprotease, einer Rübenmosaikvirusprotease, Enteropeptidase, Thrombin und Faktor Xa, und wobei die exogene proteolytische Spaltstelle im Scharnierbereich liegt; und
b) Inkubieren der Zellen und des an der Oberfläche gebundenen Antikörpers unter Bedingungen, die eine Aktivierung der Zielzellen ermöglichen.

5. Verfahren nach Anspruch 4, ferner umfassend:
c) Trennen der an die Oberfläche gebundenen Zielzellen von Zellen, die nicht an die Oberfläche gebunden sind;
optional ferner umfassend d) Kontaktieren der an die Oberfläche gebundenen Zielzellen mit einer Protease oder einem Teil einer Protease, die für die Spaltstelle spezifisch ist, um die Zielzellen von der Oberfläche zu lösen; und optional ferner umfassend e) Sammeln der aktivierten Zielzellen, die von der Oberfläche gelöst sind.

6. Verfahren nach Anspruch 3, worin die feste Oberfläche ein Bead ist, umfassend:
a) Kontaktieren einer Mischzellpopulation mit einem an Beads gebundenen Antikörper, wobei der Antikörper spezifisch an die Zielzelle in der Population bindet und wobei der Antikörper eine exogene Proteasespaltstelle für eine Protease umfasst, ausgewählt aus der Gruppe bestehend aus einer Tabakätzvirus-(TEV)-Protease, einer Rhinovirus-3C-Protease, einer TVMV-Protease, einer Pflaumenpockenvirusprotease, einer Rübenmosaikvirusprotease, Enteropeptidase, Thrombin und Faktor Xa, und wobei die exogene proteolytische Spaltstelle im Scharnierbereich liegt;
b) Trennen der an die Beads gebundenen Zielzellen von Zellen, die nicht an die Beads gebunden sind;
c) Kontaktieren der an die Beads gebundenen Zielzellen mit einer Protease oder einem Teil einer Protease, die für die Spaltstelle spezifisch ist, um die Zielzellen von den Beads zu lösen;
und
d) Sammeln der von den Beads abgetrennten Zielzellen, um eine Bead-freie Population von Zielzellen zu bilden.

7. Verfahren nach einem der Ansprüche 1, 3, 5 und 6, wobei die Protease nicht durch die Zielzelle exprimiert wird.

8. Verfahren nach einem der Ansprüche 1-6, wobei sich die Spaltstelle im oberen Scharnierbereich des Antikörpers befindet.

9. Verfahren nach einem der Ansprüche 2-6, wobei die Zellpopulation Stammzellen oder Blutzellen umfasst, optional wobei die Blutzellen mononukleäre Zellen des peripheren Blutes sind.

10. Verfahren nach einem der Ansprüche 1-6, worin die Zielzelle eine T-Zelle, eine Stammzelle oder wobei die Zielzelle CD34+ ist.

11. Verfahren nach einem der Ansprüche 1-6, worin der Antikörper spezifisch an ein endogenes Protein auf der Zielzelloberfläche oder an ein exogenes Protein auf der Zielzelloberfläche bindet.

12. Verfahren nach Anspruch 11, wobei der Antikörper spezifisch ein CD3-Epitop, ein Tra-1-60-Epitop oder ein CD34-Epitop bindet.

13. Verfahren nach einem der Ansprüche 1-6, wobei der Antikörper indirekt an die feste Oberfläche gebunden ist, vorzugsweise worin die feste Oberfläche ein Bead ist, optional wobei das Bead paramagnetisch ist.

14. Verfahren nach Anspruch 4, worin die Zielzelle eine T-Zelle ist und der Antikörper ein Anti-CD3-Antikörper ist und optional, wobei das Inkubieren von (b) weiterhin IL-2 umfasst.

## Revendications

1. Procédé destiné à détacher une cellule cible fixée à une surface solide recouverte d'anticorps recombinants comprenant : la mise en contact de la cellule cible fixée à la surface au moyen d'une protéase, ou d'une partie d'une protéase, spécifique à un site de clivage protéolytique exogène dans l'anticorps, détachant de ce fait la cellule cible de la surface, l'anticorps recombinant comprenant un site de clivage protéolytique exogène pour une protéase choisie dans l'ensemble constitué de : une protéase du virus de la gravure du tabac (TEV), une protéase 3C du rhinovirus, une protéase du virus de la marbrure nervaire du tabac (TVMV), une protéase du potyvirus de la sharka du prunier, une protéase du virus de la mosaïque du navet, entéropeptidase, thrombine et facteur Xa, et le site de clivage protéolytique exogène étant dans la région charnière.

2. Procédé destiné à isoler une cellule cible d'une population de cellules hétérogènes, comprenant :
a) la mise en contact d'une population de cellules hétérogènes avec un anticorps fixé à une surface solide, l'anticorps se liant spécifiquement à la cellule cible dans la population et l'anticorps comprenant un site de clivage protéolytique exogène pour une protéase choisie dans l'ensemble constitué de : une protéase du virus de la gravure du tabac (TEV), une protéase 3C du rhinovirus, une protéase du virus de la marbrure nervaire du tabac (TVMV), une protéase du potyvirus de la sharka du prunier, une protéase du virus de la mosaïque du navet, entéropeptidase, thrombine et Facteur Xa, et le site de clivage protéolytique exogène étant dans la région charnière.

3. Procédé selon la revendication 2, comprenant en outre :
b) la séparation des cellules cibles liées à la surface des cellules non liées à la surface ;
comprenant en outre éventuellement c) la mise en contact des cellules cibles liées à la surface au moyen d'une protéase, ou d'une partie d'une protéase, spécifique au site du clivage pour détacher les cellules cibles de la surface ; et
comprenant en outre éventuellement d) la collecte des cellules cibles détachées de la surface.

4. Procédé destiné à produire une population de cellules cibles activées, comprenant :
a) la mise en contact d'une population de cellules comprenant les cellules cibles avec un anticorps agonistique attaché à une surface solide, l'anticorps se liant spécifiquement aux cellules cibles dans la population et l'anticorps comprenant un site de clivage protéolytique exogène pour une protéase choisie dans l'ensemble constitué de : une protéase du virus de la gravure du tabac (TEV), une protéase 3C du rhinovirus, une protéase du virus de la marbrure nervaire du tabac (TVMV), une protéase du potyvirus de la sharka du prunier, une protéase du virus de la mosaïque du navet, entéropeptidase, thrombine et Facteur Xa, et le site de clivage protéolytique exogène étant dans la région charnière ; et
b) l'incubation des cellules et de l'anticorps fixé à la surface dans des conditions permettant l'activation des cellules cibles.

5. Procédé selon la revendication 4, comprenant en outre :
c) la séparation des cellules cibles liées à la surface des cellules non liées à la surface ;
comprenant en outre éventuellement d) la mise en contact des cellules cibles liées à la surface au moyen d'une protéase, ou d'une partie d'une protéase, spécifique au site du clivage pour détacher les cellules cibles de la surface ; et comprenant en outre éventuellement e) la collecte des cellules cibles activées détachées de la surface.

6. Procédé selon la revendication 3, dans lequel la surface solide est une bille comprenant :
a) la mise en contact d'une population de cellules hétérogènes avec un anticorps fixé aux billes, l'anticorps se liant spécifiquement à la cellule cible dans la population et l'anticorps comprenant un site de clivage de protéase exogène pour une protéase choisie dans l'ensemble constitué de : une protéase du virus de la gravure du tabac (TEV), une protéase 3C du rhinovirus, une protéase du virus de la marbrure nervaire du tabac (TVMV), une protéase du potyvirus de la sharka du prunier, une protéase du virus de la mosaïque du navet, entéropeptidase, thrombine et Facteur Xa, et le site de clivage protéolytique exogène étant dans la région charnière ;
b) la séparation des cellules cibles liées aux billes des cellules non liées aux billes ;
c) la mise en contact des cellules cibles liées aux billes au moyen d'une protéase, ou d'une partie d'une protéase, spécifique au site de clivage pour détacher les cellules cibles des billes ; et
d) la collecte des cellules cibles détachées des billes pour former une population de cellules cibles dépourvues de billes.

7. Procédé selon l'une quelconque des revendications 1, 3, 5 et 6, dans lequel la protéase n'est pas exprimée par la cellule cible.

8. Procédé selon l'une quelconque des revendications 1 à 6, dans lequel le site de clivage se situe dans la région charnière supérieure de l'anticorps.

9. Procédé selon l'une quelconque des revendications 2 à 6, dans lequel la population de cellules comprend des cellules souches ou comprend des cellules sanguines, éventuellement les cellules sanguines étant des cellules mononucléaires sanguines périphériques.

10. Procédé selon l'une quelconque des revendications 1 à 6, dans lequel la cellule cible est une cellule T, une cellule souche ou dans laquelle la cellule cible étant une cellule CD34 positive.

11. Procédé selon l'une quelconque des revendications 1 à 6, dans lequel l'anticorps se lie spécifiquement à une protéine endogène sur la surface de la cellule cible ou à une protéine exogène sur la surface de la cellule cible.

12. Procédé selon la revendication 11, dans lequel l'anticorps se lie spécifiquement à un épitope CD3, à un épitope Tra-1-60 ou à un épitope CD34.

13. Procédé selon l'une quelconque des revendications 1 à 6, dans lequel l'anticorps est indirectement fixé à la surface solide, la surface solide étant de préférence une bille, éventuellement la bille étant paramagnétique.

14. Procédé selon la revendication 4, dans lequel la cellule cible est une cellule T et l'anticorps est un anticorps anti-CD3 et éventuellement, l'incubation de (b) comprenant en outre l'interleukine 2 (IL-2).


### SEQUENCE LISTING

<110> SCHJETNE, Karoline AARVAK, Tanja NORDERHAUG, Lars
<120> RECOMBINANT ANTIBODY COMPOSITIONS AND
   METHODS OF USE THEREOF
<130> LT00692 PCT
<150> 61/650,388
   <151> 2012-05-22
<160> 23
<170> FastSEQ for Windows Version 4.0
<210> 1
   <211> 5
   <212> PRT
   <213> Artificial Sequence
<220>
   <223> Synthetic construct
<400> 1
   Asp Asp Asp Asp Lys
    1               5
<210> 2
   <211> 6
   <212> PRT
   <213> Artificial Sequence
<220>
   <223> Synthetic construct
<400> 2
   Leu Val Pro Arg Gly Ser
    1               5
<210> 3
   <211> 7
   <212> PRT
   <213> Artificial Sequence
<220>
   <223> sythetic contruct
<400> 3
   Glu Asn Leu Tyr Phe Gln Gly
    1               5
<210> 4
   <211> 8
   <212> PRT
   <213> Artificial Sequence
<220>
   <223> Synthetic construct
<400> 4
   Leu Glu Val Leu Phe Gln Gly Pro
   1 5
<210> 5
   <211> 7
   <212> PRT
   <213> Artificial Sequence
<220>
   <223> Synthetic construct
<220>
   <221> VARIANT
   <222> 7
   <223> Xaa=Ser or Gly
<220>
   <221> VARIANT
   <222> 2, 3, 5,
   <223> Xaa = Any Amino Acid
<400> 5
   Glu Xaa Xaa Tyr Xaa Gln Xaa
   1 5
<210> 6
   <211> 35
   <212> DNA
   <213> Artificial Sequence
<220>
   <223> synthetic primer
<400> 6
   gtaagctttc ggggacatgg gaatgcaaaa gtagc 35
<210> 7
   <211> 31
   <212> DNA
   <213> Artificial Sequence
<220>
   <223> synthetic primer
<400> 7
   gtggatccgg gctcaaacca tggagacccc t 31
<210> 8
   <211> 39
   <212> DNA
   <213> Artificial Sequence
<220>
   <223> sythetic primer
<400> 8
   aagctttcgg ggacatggaa atgcaaaaga gcggccttc 39
<210> 9
   <211> 49
   <212> DNA
   <213> Artificial Sequence
<220>
   <223> sythetic primer
<400> 9
   cagtgcccag ggatgaaaac ctgtattttc agggctgtgg ttgtaagcc 49
<210> 10
   <211> 19
   <212> PRT
   <213> Artificial Sequence
<220>
   <223> sythetic construct
<400> 10
   Pro Arg Asp Glu Asn Leu Tyr Phe Gln Gly Cys Gly Cys Lys Pro Cys
    1               5                  10                  15
   Ile Cys Thr
<210> 11
   <211> 29
   <212> DNA
   <213> Artificial Sequence
<220>
   <223> sythetic primer
<400> 11
   ttataagctt cttaccttat gtgcttgtg 29
<210> 12
   <211> 31
   <212> DNA
   <213> Artificial Sequence
<220>
   <223> sythetic primer
<400> 12
   atatggatcc tttgtctcta acactcattc c 31
<210> 13
   <211> 21
   <212> DNA
   <213> Artificial Sequence
<220>
   <223> sythetic primer
<400> 13
   tgctgtcttt gctgtcctga t 21
<210> 14
   <211> 21
   <212> DNA
   <213> Artificial Sequence
<220>
   <223> sythetic primer
<400> 14
   tagaggtcag actgcaggac a 21
<210> 15
   <211> 21
   <212> DNA
   <213> Artificial Sequence
<220>
   <223> sythetic primer
<400> 15
   caagaagcac acgactgagg c 21
<210> 16
   <211> 23
   <212> DNA
   <213> Artificial Sequence
<220>
   <223> sythetic primer
<400> 16
   cttgaccagg catcctagag tca 23
<210> 17
   <211> 37
   <212> DNA
   <213> Artificial Sequence
<220>
   <223> sythetic primer
<400> 17
   attctgcatt ccaaattgtt ctcacccagt ctccagc 37
<210> 18
   <211> 41
   <212> DNA
   <213> Artificial Sequence
<220>
   <223> sythetic primer
<400> 18
   tcgacgtacg ttctactcac gtttcagctc cagcttggtc c 41
<210> 19
   <211> 27
   <212> DNA
   <213> Artificial Sequence
<220>
   <223> sythetic primer
<400> 19
   tctgcattcc aggtccagct gcagcag 27
<210> 20
   <211> 39
   <212> DNA
   <213> Artificial Sequence
<220>
   <223> sythetic primer
<400> 20
   gacgtacgtt ctactcacct gaggagacgg tgactgacc 39
<210> 21
   <211> 19
   <212> DNA
   <213> Artificial Sequence
<220>
   <223> sythetic primer
<400> 21
   ccaagctagc ttggtaccg 19
<210> 22
   <211> 20
   <212> DNA
   <213> Artificial Sequence
<220>
   <223> sythetic primer
<400> 22
   ggatcctttg tctctaacac 20
<210> 23
   <211> 19
   <212> DNA
   <213> Artificial Sequence
<220>
   <223> sythetic primer
<400> 23
   gaattcgccc ttgtggatc 19